(19) Europäisches Patentamt
European Patent Office
Office européen des brevets

(11) Veröffentlichungsnummer: **0 207 483 B1**

# (12) EUROPÄISCHE PATENTSCHRIFT

(45) Veröffentlichungstag der Patentschrift: **18.03.92**

(21) Anmeldenummer: **86108886.2**

(22) Anmeldetag: **30.06.86**

Die Akte enthält technische Angaben, die nach dem Eingang der Anmeldung eingereicht wurden und die nicht in dieser Patentschrift enthalten sind.

(51) Int. Cl.5: **C07D 498/04**, C07D 513/04, C07D 487/04, A61K 31/41, C07D 263/58, C07D 417/12, C07D 413/12, //(C07D487/04, 235:00,225:00),(C07D498/04, 265:00,235:00),(C07D513/04, 277:00,235:00)

## (54) Neue Benzimidazole, Verfahren zu ihrer Herstellung und diese Verbindungen enthaltende Arzneimittel sowie Zwischenprodukte.

(30) Priorität: **05.07.85 DE 3524067**

(43) Veröffentlichungstag der Anmeldung: **07.01.87 Patentblatt 87/02**

(45) Bekanntmachung des Hinweises auf die Patenterteilung: **18.03.92 Patentblatt 92/12**

(84) Benannte Vertragsstaaten: **AT BE CH DE FR GB IT LI LU NL SE**

(56) Entgegenhaltungen:
**EP-A- 0 039 818**
**EP-A- 0 161 632**
**DE-A- 1 694 078**
**GB-A- 0 270 606**
**US-A- 4 415 572**

(73) Patentinhaber: **BOEHRINGER MANNHEIM GMBH**
**Sandhofer Strasse 116**
**W-6800 Mannheim 31(DE)**

(72) Erfinder: **von der Saal, Wolfgang, Dr. rer. nat.**
**Neuer Burgweg 3**
**W-6940 Weinheim(DE)**
Erfinder: **Mertens, Alfred, Dr.**
**In der Schanz 31**
**W-6905 Schriesheim(DE)**
Erfinder: **Berger, Herbert, Dr. phil.**
**Völklinger Strasse 16**
**W-6800 Mannheim-31(DE)**
Erfinder: **Müller-Beckmann, Bernd, Dr.**
**Hochgewanne 46**
**W-6718 Grünstadt(DE)**

**EP 0 207 483 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 82, 23. Juni 1975, Seite 547, Zusammenfassung Nr. 170950v, Columbus, Ohio, US

Ann. Chim. (Rome) (1978) 68, 139-141

J. Het. Chem. (1968) 5, 147-148

Erfinder: Strein, Klaus, Prof. Dr. rer. nat.
Eichenstrasse 45
W-6944 Hemsbach(DE)

## Beschreibung

Die vorliegende Erfindung betrifft neue Benzimidazole der allgemeinen Formel I

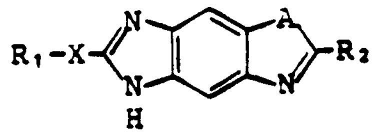

(I)

in welcher

$R_1$ den Pyrrol-, Furan-, Thiophen-, Pyrazol-, Imidazol-, Thiazol-, Isothiazol-, Oxazol-, Isoxazol-, Triazol-, Tetrazol-, Thiadiazol-, Oxadiazol-, Pyridin-, N-Oxy-Pyridin-, Pyrazin-, N,N-Dioxy-pyrazin-, Pyrimidin-, N,N-Dioxypyrimidin-, Pyridazin-, Oxazin-, Thiazin-, Triazin-, Tetrazinrest darstellt, sowie deren $C_1$-$C_6$-Alkyl-, $C_1$-$C_6$-Alkoxy-, $C_1$-$C_6$-Alkylmercapto- und chlor-substituierten Derivate, oder den Phenylrest der allgemeinen Formel II,

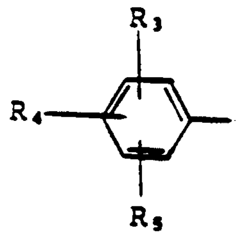

(II)

in der

$R_3$ Wasserstoff, eine Alkylsulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino oder Dialkylaminogruppe substituierte Carbonylgruppe oder eine durch eine Amino-, Dialkylamino- oder Morpholinogruppe substituierte Sulfonylgruppe, wobei jeder der vorstehend genannten Alkylteile 1 oder 2 Kohlenstoffatome enthalten kann, eine Nitro-, Cyan- oder Alkylaminosulfonylgruppe mit 1-4 Kohlenstoffatomen, eine Alkylcarbonylamino-, Aminocarbonylamino- oder N-Alkyl-aminocarbonylaminogruppe, eine Alkylmercapto-, Alkylsulfinyl- oder Alkylsulfonylgruppe, wobei jeder der vorgenannten Alkylteile 1 oder 2 Kohlenstoffatome enthalten kann, eine Halogen-, Amino-, Hydroxy-, Dialkylamino-, Alkyl-, Alkoxy-, Alkenyloxy- oder Alkinyloxygruppe vorzugsweise mit 1-3 Kohlenstoffatomen, eine Cyanmethoxy- oder Methoxycarbonylmethoxygruppe, die Trifluormethylgruppe oder die 1-Imidazolylgruppe, $R_4$ Wasserstoff, eine Alkygruppe mit 1-3 Kohlenstoffatomen, eine Alkoxy- oder Dialkylaminogruppe mit 1 oder 2 Kohlenstoffatomen in jedem Alkylteil oder ein Halogenatom, und

$R_5$ Wasserstoff oder die Methoxygruppe ist,

X ein Valenzstrich, eine $C_1$-$C_4$-Alkylengruppe oder die Vinylengruppe sein kann,

A ein Sauerstoffatom, ein Schwefelatom, oder die Gruppe N-$R^6$ bedeutet, wobei $R_6$ ein Wasserstoffatom oder eine $C_1$-$C_4$-Alkylgruppe darstellt,

$R_2$ ein Wasserstoffatom, eine $C_1$-$C_4$-Alkyl-, eine Hydroxy- oder eine Mercaptogruppe darstellt, mit Ausnahme der Verbindung 2-(4-Aminophenyl)-1,7-dihydrobenzo-[1,2-d:4,5-d']bisimidazol, deren Tautomere und deren physiologisch verträgliche Salze anorganischer und organischer Säuren und Verfahren zu ihrer Herstellung, sowie diese Verbindungen enthaltende Arzneimittel.

Für den Fall, daß Verbindungen der allgemeinen Formel I hergestellt wurden, die ein asymmetrisches Atom enthalten, sind auch Gegenstand der Erfindung die optisch aktiven Formen und racemischen Gemische dieser Verbindungen.

Diese neuen Verbindungen der vorliegenden Erfindung weisen wertvolle pharmakologische Eigenschaften auf, insbesondere steigern sie die Herzkraft und/oder wirken blutdrucksenkend und/oder beeinflussen die Thrombozytenfunktion und verbessern die Mikrozirkulation.

Verbindungen dieser Art mit der allgemeinen Formel I sind zwar aus der DE-A-1 694 078 und aus zwei Veröffentlichungen [Ann. Chim. (Rome) (1978) 68, 139-141, Chem. Abstr. (1979) 90, 105583d und J. Heterocycl. Chem. (1968) 5, 147-148, D6, Chem. Abstr. (1968) 68, 78263m] bekannt, jedoch weisen sie

nicht die gewünschten pharmakologischen Eigenschaften auf.

In der älteren EP-A- 0 161 632 werden Verbindungen und Zwischenprodukte beschrieben, welche eine tricyklische bzw. bicyclische Struktur mit einem zentralen 6-Ring und symmetrisch dazu zwei heterocyklischen 5-Ringen bzw. mit einem 6-Ring und einem heterocyklischen 5-Ring aufweisen. Darüber hinaus sind jedoch beachtliche strukturelle Unterschiede zu den in der Anmeldung beschriebenen Verbindungen und Zwischenprodukten vorhanden. Für die in der EP-A-0 161 632 beschriebenen Verbindungen werden dieselben pharmakologischen Wirkungen angegeben wie für die in der vorliegenden Anmeldung beschriebenen.

Alkyl-, Alkoxy- und Alkylmercapto-Substituenten in den heterocyklischen Fünf- und Sechsringen sind bevorzugt der Methyl-, Ethyl-, Methoxy-, Ethoxy-, Methylmercapto- und Ethylmercaptorest. Unter Halogen ist Fluor, Chlor und Brom, vorzugsweise Chlor zu verstehen.

Bedeutet $R_1$ einen Phenylring der allgemeinen Formel II, so sind die bei $R_3$, $R_4$ und $R_5$ genannten Substituenten bevorzugt Gruppen wie beispielsweise die Methansulfonyloxy-, Ethansulfonyloxy-, n-Propansulfonyloxy-, Isopropansulfonyloxy-, Trifluormethansulfonyloxy-, Methylsulfenylmethyl-, Ethylsulfenylmethyl-, n-Propylsulfenylmethyl-, Methylsulfinylmethyl-, Ethylsulfinylmethyl-, n-Propylsulfinylmethyl-, Methylsulfonylmethyl-, Ethylsulfonylmethyl-, n-Propylsulfonylmethyl-, Methansulfonylamino-, Ethansulfonylamino-, n-Propansulfonylamino-, Trifluormethansulfonylamino-, N-Methyl-methansulfonylamino-, N-Ethyl-methansulfonylamino-, N-Methyl-ethansulfonylamino-, N-Ethyl-ethansulfonylamino-, N-Isopropyl-ethansulfonylamino-, N-Methyl-n-propansulfonylamino-, N-Ethyl-n-propansulfonylamino-, N-n-Propyl-n-propansulfonylamino-, N-Methyl-trifluormethansulfonylamino-, N-Ethyltrifluormethansulfonylamino, N-Isopropyl-trifluormethansulfonylamino-, Methoxycarbonyl-, Ethoxycarbonyl-, Propoxycarbonyl-, Isopropoxycarbonyl-, Methylaminocarbonyl-, Ethylaminocarbonyl-, Dimethylaminocarbonyl-, Di-n-propylaminocarbonyl-, N-Methyl-ethylaminocarbonyl-, Trifluormethyl-, Methylaminosulfonyl-, Ethylaminosulfonyl-, n-Propylaminosulfonyl-, n-Butylaminosulfonyl-, n-Pentylaminosulfonyl-, Dimethylaminosulfonyl-, Diethylaminosulfonyl-, Di-n-propylaminosulfonyl-, N-Methyl-isopropylaminosulfonyl-, Acetylamino-, Propionylamino-, Methylaminocarbonylamino-, Ethylaminocarbonylamino-, Propylaminocarbonylaminogruppe, eine Methyl-, Ethyl-, Propyl-, Methoxy-, Ethoxy-, Propoxygruppe, eine Allyloxy-, 2-Butenyloxy-, 3-Butenyloxy-, 2-Pentenyloxy-, Propargyloxy-, 2-Butinyloxy-, 3-Butinyloxy, Cyanmethyloxy-, Cyanethyloxy-, Methoxycarbonylmethoxy-, Methoxycarbonylethoxygruppe, eine Methylmercapto-, Ethylmercapto-, eine Methylsulfinyl-, Ethylsulfinyl-, Methylsulfonyl- und Ethylsulfonylgruppe, eine Dimethylaminocarbonylamino-, Diethylaminocarbonylamino-,N-Methyl-methylcarbonylamino, N-Ethylmethylcarbonylamino-, N-Methyl-ethylcarbonylamino- und N-Ethylethylcarbonylaminogruppe, eine Methoxysulfonyl- und Ethoxysulfonylgruppe.

Bei Sulfonyl- und Carbonylgruppen, die durch cyclische Iminogruppen substituiert sein koennen, sind bevorzugt die Morpholino-, Pyrrolidino-, Piperidino- und Hexamethyleniminosulfonylgruppen.

Der Phenylteil kann 1 bis 3 der genannten Substituenten tragen.

Bevorzugte monosubstituierte Verbindungen sind die Hydroxy-, $C_1$-$C_3$ Alkyl-, Trifluormethyl-, $C_1$-$C_3$-Alkoxy-, Allyloxy-, Propargyloxy-, Cyanmethyloxy-, Methoxycarbonylmethoxy-, Halogen-, Nitro-, Amino-, Aminocarbonyl-, Methoxycarbonyl-, $C_1$-$C_3$ Dialkylamino-, $C_1$-$C_3$ Alkylmercapto-, $C_1$-$C_3$ Alkylsulfinyl-, $C_1$-$C_3$ Alkylsulfonyl-, $C_1$-$C_3$ Alkylsulfonyloxy- und die 1-Imidazolylverbindungen, wobei der Substituent in 2-, 3- oder 4-stellung stehen kann.

Bevorzugte disubstituierte Verbindungen enthalten als Substituenten eine Alkansulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino- oder Morpholinogruppe substituierte Sulfonylgruppe, eine Alkylaminosulfonyl-, Alkylcarbonylamino-, Aminocarbonylamino- oder N-Alkyl-aminocarbonylaminogruppe, eine Hydroxy-, Alkyl-, Alkoxy-, Allyloxy-, Propargyloxy-, Cyanmethyloxy-, Methoxycarbonylmethyloxy-, Cyan-, Halogen-, Nitro-, Amino-, Dialkylamino-, Alkylmercapto-, Alkylsulfinyl-, Alkylsulfonyl- oder eine 1-Imidazolylgruppe, wobei die beiden Substituenten gleich oder verschieden sein koennen und in 2,3- 2,4-, 2,5-, 2,6-, 3,4- und 3,5-Stellung bevorzugt jedoch in 2,4-, 2,5-und 3,4-Stellung stehen koennen und die vorgenannten Alkylreste, allein oder in Kombination mit anderen Resten, 1-3 C-Atome aufweisen koennen. Bevorzugter trisubstituierter Phenylrest ist 3,4,5-Trimethoxyphenyl.

Fuer X ist der Valenzstrich, eine Methylen-, Ethylen- oder die Vinylengruppe bevorzugt.

Fuer A ist bevorzugt ein Sauerstoffatom, ein Schwefelatom oder eine Gruppe

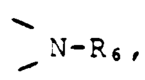

in der fuer $R_6$ Wasserstoff, die Methyl-, Ethyl-, Propyl-, Isopropyl-, Butyl-, 2-Butyl-, und 2-(2-Methyl)-propylgruppe bevorzugt ist.

Fuer $R_2$ sind bevorzugt eine Wasserstoffatom, die Methyl-, Ethyl-, Propyl-, Butyl-, 2-Propyl-, 2-Butyl- und die 2-(2-Methyl)propylgruppe, die Hydroxy- oder Mercaptogruppe.

Besonders bevorzugte Verbindungen sind Verbindungen der allgemeinen Formel I, in der

- $R_1$ den Pyrrol-, Furan-, Thiophen-, Pyrazol-, Imidazol-, Thiazol-, Isothiazol-, Oxazol-, Isoxazol-, Triazol-, Tetrazol-, Thiadiazol-, Oxadiazol-, Pyridin-, N-Oxy-Pyridin-, Pyrazin-, N,N-Dioxy-pyrazin-, Pyrimidin-, N,N-Dioxypyrimidin-, Pyridazin-, Oxazin-, Thiazin-, Triazin-, Tetrazinrest darstellt, sowie deren Methyl-, Ethyl-, Methoxy-, Ethoxy-, Methylmercapto-, Ethylmercapto- und chlorsubstituierten Derivate,
  oder den Phenylrest der allgemeinen Formel II, in der
- $R_3$ ein Wasserstoff, die Methansulfonyloxy-, Trifluormethansulfonyloxy-, Methansulfonylamino-, Trifluormethansulfonylamino-, Methansulfonyl-methylamino-, Trifluormethansulfonylmethylamino-, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfonyl-, Hydroxy-, Methyl-, Methoxy-, Propargyloxy-, Cyanmethoxy-, Methoxycarbonylmethoxy-, Cyan-, Chlor-, Nitro-, Amino-, Dimethylamino-, Trifluormethyl-, oder die 1-Imidazolylgruppe darstellt,
- $R_4$ Wasserstoff, die Methyl-, Methoxy-, Dimethylamino- oder Chlorgruppe bedeutet und
- $R_5$ Wasserstoff oder die Methoxygruppe ist,
- X einen Valenzstrich, eine Alkylengruppe mit 1 oder 2 Kohlenstoffatomen oder die Vinylengruppe bedeutet,
- A ein Sauerstoff- oder Schwefelatom oder die Gruppe $NR_6$ ist, in der $R_6$ Wasserstoff, die Methyl-, Ethyl-, Propyl-, Isopropylgruppe ist,
- $R_2$ Wasserstoff, Methyl-, Ethyl-, Propyl-, Butyl-, Isopropyl-, 2-Butyl-, tert.-Butyl-, Hydroxy- oder Mercaptogruppe ist.

Die Verbindungen der allgemeinen Formel I koennen nach den folgenden Schemata 1-4 hergestellt werden.

Wie aus Schema 1 ersichtlich kann man Verbindungen der allgemeinen Formel III und VI durch Nitrierung in Verbindungen der allgemeinen Formel X und XI ueberfuehren, welche nach Reduktion der Nitrogruppe und Ringschluß die Verbindungen der allgemeinen Formel I' liefern, wobei A, X und $R_2$ die oben angegebenen Bedeutungen besitzen und in welcher

- $R_1'$ einen heterocyclischen Fuenfring mit 1-4 Heteroatomen oder einen heterocyclischen Sechsring mit 1-5 Heteroatomen darstellt, wobei die Heteroatome der vorgenannten Fuenf- und Sechsringe gleich oder verschieden sein koennen und Stickstoff, Sauerstoff oder Schwefel bedeuten und gegebenenfalls an einem oder mehreren Stickstoffatomen ein Sauerstoffatom tragen koennen, und die vorgenannten Fuenf- und Sechsringe gegebenenfalls durch eine oder mehrere Alkyl-, Alkoxy-, Alkylmercapto-, Hydroxy-, Nitro-, Amino-, Halogen- oder Cyangruppen substituiert sein koennen,
  oder einen Phenylring der allgemeinen Formel II darstellt,

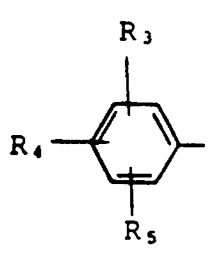

(II),

wobei $R_3$, $R_4$ und $R_5$ gleich oder verschieden sein koennen und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyl-trifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino-, Dialkylamino- oder cyclische Iminogruppe substituierte Sulfonylgruppe, wobei eine Methylengruppe durch ein Schwefel- oder Sauerstoffatom ersetzt sein kann, eine Alkylcarbonylamino-, Aminocarbonylamino- oder Alkylaminocarbonylaminogruppe, eine Alkylmercapto-, Alkylsulfinyl- oder Alkylsulfonylgruppe,

Schema 1

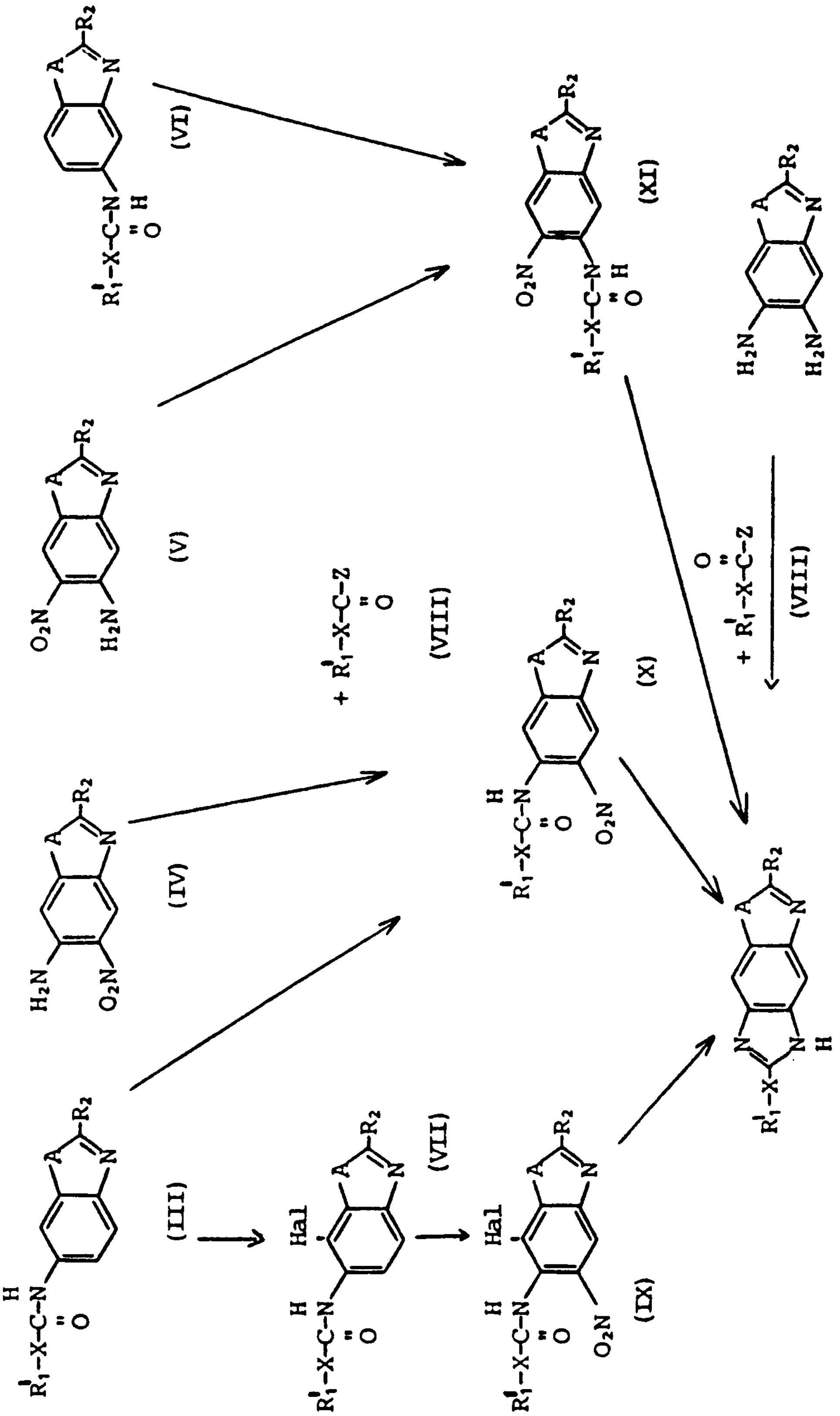


eine Alkenyloxy-, Alkinyloxy-, Cyanalkoxy-, Carboxyalkoxy-, Alkoxycarbonylalkoxy, eine Nitro-, Halogen-, Amino-, Hydroxy-, Alkyl-, Alkoxy-, Dialkylamino-, 1-Imidazolyl-, Trifluormethyl- oder Cyangruppe sein koennen,
oder fuer den Fall daß X eine Bindung darstellt, $R_1'$ neben den erwaehnten Gruppen auch eine Alkyl-, Alkenyl-, Alkinyl-, Cycloalkyl-, Cycloalkenyl-, Halogenalkyl-, Carboxyalkyl-,

Alkoxycarbonylalkyl- oder Alkoxyalkylgruppe bedeutet.

In manchen Faellen ist es zweckmaeßig, eine reaktive Position im Phenylteil der Verbindungen III zu schuetzen durch Herstellung der Verbindungen der allgemeinen Formel VII, wobei $R_1'$ , X, $R_2$ und A die angegebene Bedeutung besitzen und Hal eine Halogenidgruppe, vorzugsweise Bromid, darstellt. Nach Nitrierung erhaelt man Verbindungen der allg. Formel IX, die nach Reduktion der Nitrogruppe unter gleichzeitiger Abspaltung von Hal die Verbindungen der allgemeinen Formel I' liefern.

Die als Zwischenprodukte wichtigen Verbindungen X und XI koennen auch aus den Verbindungen IV und V durch Umsetzung mit Verbindungen der Formel VIII erhalten werden, wobei $R_1'$ , X, A und $R_2$ die oben angegebene Bedeutung besitzen und Z eine leicht abspaltbare Gruppe darstellt.

Verbindungen der allgemeinen Formel I' koennen schließlich auch durch direkte Umsetzung von Verbindungen der allgemeinen Formel XII mit Verbindungen der allg. Formel VIII hergestellt werden, wobei $R_1'$ , X, Z, A und $R_2$ die oben angegebenen Bedeutungen besitzen.

Insbesondere versteht man unter den Verbindungen der allgemeinen Formel VIII Aldehyde, sowie Saeurehalogenide wie Saeurechloride, Carbonsaeureester wie Methyl- und Ethylester und andere aktivierte Carbonsaeurederivate, wie z.B. Anhydride, sowie die Carbonsaeuren selbst.

Ist die Verbindung der Formel VIII ein Aldehyd findet die Umsetzung zur Schiffschen Base mit Verbindungen der allgemeinen Formel XII vorzugsweise in alkoholischem Medium statt, die anschließende Cyclisierung und Oxidation zu den Verbindungen der allgemeinen Formel I' erfolgt durch Erwaermen des Reaktionsansatzes zum Rueckfluß in Gegenwart von Luftsauerstoff und katalytischen Mengen Saeure, wie z.B. Toluolsulfonsaeure.

Ist die Verbindung der allgemeinen Formel VIII eine Carbonsaeure, so findet die Umsetzung mit Verbindungen der allgemeinen Formeln IV, V und XII zum Amid in Gegenwart eines wasserentziehenden Mittels, vorzugsweise in Polyphosphorsaeure bei Temperaturen zwischen 50 und 250 °C, vorzugsweise zwischen 100 und 200 °C statt.

Ist die Verbindung der allgemeinen Formel VIII ein Carbonsaeurederivat, findet die Umsetzung mit Verbindungen der allgemeinen Formeln IV, V und XII zum Amid in inerten Loesungsmitteln, vorzugsweise in Methylenchlorid oder Pyridin statt und die anschließende Cyclisierung zu Verbindungen der allgemeinen Formel I' wird nach vorheriger Hydrierung der Nitrogruppe in Verbindungen der Formel X und XI, in Verbindungen der Formel IX unter gleichzeitiger Abspaltung von Hal, in einem Loesungsmittel oder Loesungsmittelgemisch wie Ethanol, Isopropanol, Eisessig, Benzol, Chlorbenzol, Glycol, Diethylenglycoldimethylether, Sulfolan oder Dimethylformamid bei Temperaturen zwischen 0 ° und 250 °C, vorzugsweise jedoch bei der Siedetemperatur des Reaktionsgemisches, gegebenenfalls in Gegenwart eines Kondensationsmittels wie Phosphoroxychlorid, Thionylchlorid, p-Toluolsulfonsaeure, Salzsaeure, Schwefelsaeure, Phosphorsaeure, Polyphosphorsaeure oder gegebenenfalls auch in Gegenwart einer Base wie Natriumhydroxyd, Kaliummethylat oder Kalium-tert.-butylat durchgefuehrt. Die Cyclisierung kann jedoch auch ohne Loesungsmittel und/oder Kondensationsmittel durchgefuehrt werden.

Die oben erwaehnte Hydrierung der Nitrogruppe wird vorzugsweise in einem Loesungsmittel wie Wasser, Ethanol, Eisessig, Essigsaeureethylester oder Dimethylformamid zweckmaeßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Saeure, mit Salzen wie Eisen(II)sulfat, Zinn(II)chlorid, Natriumsulfid, Natriumhydrogensulfid oder Natriumdithionit, oder mit Hydrazin in Gegenwart von Raney-Nickel bei Temperaturen zwischen 0 und 250 °C, vorzugsweise jedoch bei Raumtemperatur, durchgefuehrt.

Die Verbindungen III, IV, V, VI und XII sind literaturbekannt oder lassen sich nach literaturbekannten Verfahren leicht herstellen. Die Verbindungen VII, IX, X und XI sind neu und ebenfalls Gegenstand der Erfindung.

Wie aus Schema 2 ersichtlich, lassen sich Verbindungen der allgemeinen Formel I'', in der $R_2$ und A die angegebenen Bedeutungen besitzen und $R_1''$ eine Hydroxy-, Mercapto-, oder Aminogruppe bedeutet, nach literaturbekannten Verfahren herstellen durch Ringschluß von Verbindungen der allgemeinen Formel XII mit Reagenzien wie Harnstoff, Phosgen, Thiophosgen, Carbonyldiimidazol oder Bromcyan.

Schema 2:

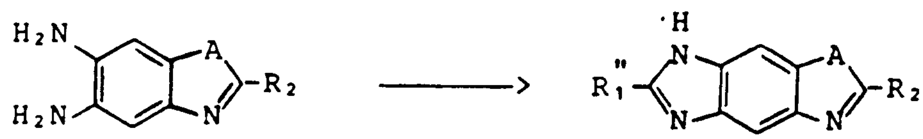

(XII) (I")

(Vergleiche hierzu: E.S. Schipper und A.R. Day in R.C. Elderfield (Ed.), Heterocyclic Compounds, Vol. 5, John Wiley and Sons, New York 1957, S. 284; J.W. Cornforth, ebenda, S. 439; J.M. Sprague, A.H. Land, ebenda, S. 548).

Diese Reaktionen werden vorzugsweise in einem Loesungsmittel oder Loesungsmittelgemisch wie Benzol, Toluol, Chlorbenzol, Dimethylformamid, Methylenchlorid oder waessriger Salzsaeure bei Temperaturen zwischen -20 °C und 100 °C, vorzugsweise bei Raumtemperatur vorgenommen.

Die in Schema 2 dargestellten Ringschlueße sind gleichermaßen auch auf Verbindungen der allgemeinen Formel XIII anwendbar, wobei Verbindungen der allgemeinen Formel I''' entstehen, in denen $R_1$, X und A die oben angegebenen Bedeutungen besitzen und $R_2'$ eine Hydroxy-, Mercapto- oder Aminogruppe bedeutet (Schema 3):

Schema 3:

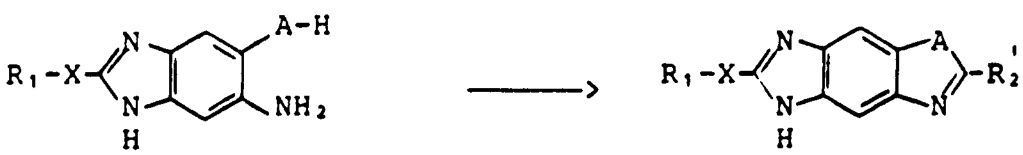

(XIII) (I"')

Wie aus Schema 4 ersichtlich, lassen sich Verbindungen der allgemeinen Formel I''', in der $R_1$, X und A die angegebenen Bedeutungen besitzen und $R_2''$ ein Wasserstoffatom oder eine Alkylgruppe bedeutet, herstellen durch Umsetzung von Verbindungen der allgemeinen Formel XIII mit Verbindungen der allgemeinen Formel XIV, in der $R_2''$ ein Wasserstoffatom oder eine Alkylgruppe und Y einen leicht abspaltbaren Rest darstellt.

(Vergleiche: E.S. Schipper und A.R. Day, in R.C. Elderfield (Ed.), Heterocyclic Compounds, Vol. 5, J. Wiley and Sons, N.Y. 1957, S. 274; J.W. Cornforth, ebenda S. 418; J.M. Sprague, A.H. Land, ebenda, S. 506).

Schema 4:

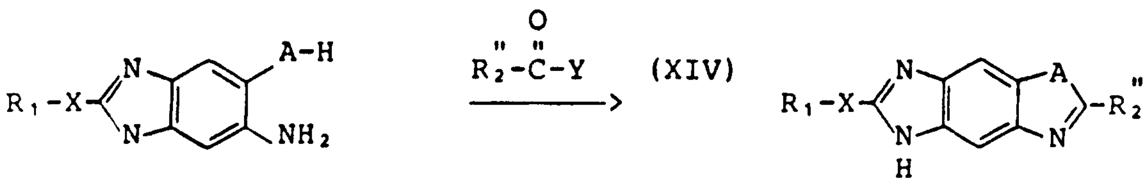

(XIV)

(XIII)

(I'''')

Verbindungen der allgemeinen Formel I koennen auch nachtraeglich in eine andere Verbindungen der Formel I umgewandelt werden. Dies trifft zum Beispiel zu

a) fuer die Oxidation eines Fuenf- oder Sechsringes mit einem oder mehreren Stickstoffatomen zu den entsprechenden N-Oxiden. Die Oxidation wird zweckmaeßig mit einem oder mehreren Aequivalenten des verwendeten Oxidationsmittels durchgefuehrt, z.B. mit Wasserstoffperoxid in Eisessig, Trifluoressigsaeure oder in Ameisensaeure bei 20 - 100°C oder in Aceton bei 0 - 60°C, mit einer Persaeure wie Perameisensaeure oder m-Chlorperbenzoesaeure in Eisessig, Trifluoressigsaeure, Methylenchlorid oder Chloroform bei Temperaturen zwischen 0 und 60°C.

b) fuer die Hydrierung einer Vinylenverbindung (X = -CH=CH-) in eine entsprechende Ethylenverbindung (X = $-CH_2-CH_2-$). Die Hydrierung wird vorzugsweise in einem Loesungsmittel wie Wasser, Wasser/Ethanol, Methanol, Eisessig, Essigsaeureethylester oder Dimethylformamid zweckmaeßigerweise mit Wasserstoff in Gegenwart eines Hydrierungskatalysators wie Raney-Nickel, Platin oder Palladium/Kohle durchgefuehrt.

c) fuer die Herstellung von Verbindungen der allgemeinen Formel I, in der $R_3$ eine Alkylsulfinyl-, Alkylsulfonyl-, Alkylsulfinylmethyl- oder Alkylsulfonylmethylgruppe darstellt, durch nachtraegliche Oxidation einer Verbindung der allgemeinen Formel XVI

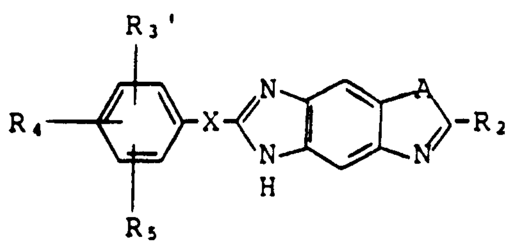

(XVI),

in der

$R_2$, A, $R_4$, $R_5$ und X wie eingangs definiert sind und $R_3$' eine Alkylmercapto-, Alkylsulfinyl-, Alkylsulfinylmethyl- oder Alkylsulfenylmethylgruppe mit jeweils 1-3 Kohlenstoffatomen im Alkylteil ist.

Die Oxidation wird vorzugsweise in einem Loesungsmittel oder Loesungsmittelgemisch, z.B. in Wasser, Wasser/Pyridin, Aceton, Eisessig, verduennter Schwefelsaeure oder Trifluoressigsaeure, je nach dem verwendeten Oxidationsmittel zweckmaeßigerweise bei Temperaturen zwischen -80 und 100°C durchgefuehrt.

Zur Herstellung einer Alkylsulfinyl- oder Alkylsulfinylmethylverbindung der allgemeinen Formel I wird die Oxidation zweckmaeßigerweise mit einem Aequivalent des verwendeten Oxidationsmittel durchgefuehrt, z.B. mit Wasserstoffperoxid in Eisessig, Trifluoressigsaeure oder Ameisensaeure bei 0 bis 20°C oder in Aceton bei 0 bis 60°C, mit einer Persaeure wie Perameisensaeure in Eisessig oder Trifluoressigsaeure bei 0 bis 50°C oder mit m-Chlorperbenzoesaeure in Methylenchlorid oder Chloroform bei -20 bis 60°C, mit Natriummetaperjodat in waessrigem Methanol oder Ethanol bei -15 bis 25°C, mit Brom in Eisessig oder waessriger Essigsaeure, mit N-Brom-succinimid in Ethanol, mit tert.-Butylhypochlorit in Methanol bei -80° bis -30°C, mit Jodbenzodichlorid in waessrigem Pyridin bei 0 bis 50°C, mit Salpetersaeure in Eisessig bei 0 bis 20°C, mit Chromsaeure in Eisessig oder in Aceton bei 0 bis 20°C und mit Sulfurylchlorid in Methylenchlorid bei -70°C, der hierbei erhaltene Thioether-Chlor-Komplex wird zweckmaeßigerweise mit waessrigem Ethanol hydrolysiert.

Zur Herstellung einer Alkylsulfonyl- oder Alkylsulfonylmethylverbindung der allgemeinen Formel I wird die Oxidation zweckmaeßigerweise mit einem bzw. mit zwei oder mehr Aequivalenten des verwendeten Oxidationsmittels durchgefuehrt, z.B. Wasserstoffperoxid in Eisessig, Trifluoressigsaeure oder in Ameisensaeure bei 20 bis 100°C oder in Aceton bei 0 bis 60°C, mit einer Persaeure wie Perameisensaeure oder m-Chlorperbenzoesaeure in Eisessig, Trifluoressigsaeure, Methylenchlorid oder Chloroform bei Temperaturen zwischen 0 und 60°C, mit Salpetersaeure in Eisessig bei 0 bis 20°C, mit Chromsaeure oder Kaliumpermanganat in Eisessig, Wasser/Schwefelsaeure oder in Aceton bei 0 bis 20°C.

Die Herstellung von Verbindungen der allgemeinen Formel I, in der $R_3$ eine Alkansulfonyloxy-, Trifluormethansulfonyloxy-, Alkansulfonylamino-, N-Alkyl-alkansulfonylamino-, Trifluormethansulfonylamino-, oder N-Alkyl-trifluormethansulfonylaminogruppe darstellt, wird erreicht durch die nachtraegliche Umsetzung einer Verbindung der allgemeinen Formel XVII

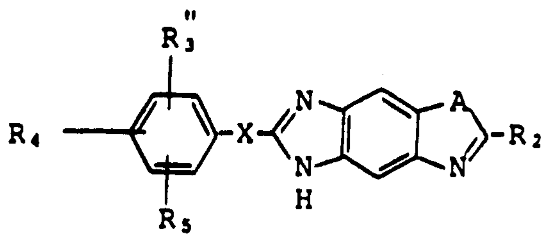

(XVII),

in der

$R_2$, A, $R_4$, $R_5$ und X wie eingangs definiert sind und $R_3''$ eine Hydroxy-, Amino- oder N-Alkylaminogruppe mit 1-3 Kohlenstoffatomen im Alkylteil darstellt, mit einer Sulfonsaeure der allgemeinen Formel XVIII

$R_7$-$SO_2OH$ (XVIII),

in der

$R_7$ eine Alkylgruppe mit 1-3 Kohlenstoffatomen oder eine Trifluormethylgruppe darstellt, in Gegenwart eines wasserentziehenden und/oder die Saeure oder das Amin aktivierenden Mittels oder mit deren reaktionsfaehigen Derivaten.

Die Umsetzung wird zweckmaeßigerweise in einem Loesungsmittel oder Loesungsmittelgemisch wie Methylenchlorid, Ether, Tetrahydrofuran, Dioxan oder Benzol gegebenenfalls in Gegenwart eines saeurebindenden Mittels wie Natriumcarbonat, Triethylamin oder Pyridin, wobei die beiden letzteren gleichzeitig auch als Loesungsmittel verwendet werden koennen, in Gegenwart eines die Saeure aktivierenden oder wasserentziehenden Mittels wie Thionylchlorid oder Phosphorpentachlorid, vorzugsweise jedoch mit einem reaktionsfaehigen Derivat einer Verbindung der allgemeinen Formel XVIII, z.B. mit deren Anhydrid oder Halogenid wie Methansulfonsaeurechlorid oder Ethansulfonsaeurechlorid, vorzugsweise bei Temperaturen zwischen 0 und 100°C, z.B. bei Temperaturen zwischen Raumtemperatur und 50°C, durchgefuehrt.

Die Herstellung von Verbindungen der allgemeinen Formel I, in der $R_3$ eine durch eine Amino-, Alkylamino-, Dialkylaminooder Hydrazinogruppe substituierte Carbonyl- oder Sulfonylgruppe darstellt, wird erreicht durch die nachtraegliche Umsetzung einer Verbindung der allgemeinen Formel XIX

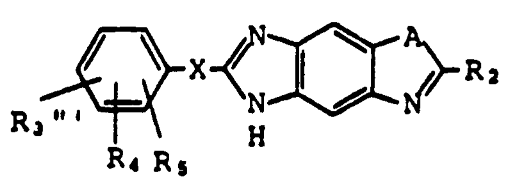

(XIX),

in der

$R_2$, $R_4$, $R_5$, A und X wie eingangs definiert sind und $R_3'''$ eine Carboxyl- oder Hydroxysulfonylgruppe darstellt, oder ein reaktionsfaehiges Derivat hiervon wie z.B. Ester oder Saeurechlorid mit Hydrazin, einem cyclischen Amin oder einem Amin der allgemeinen Formel XX

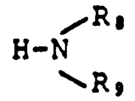

(XX) ,

in der

$R_8$ und $R_9$, die gleich oder verschieden sein koennen, Wasserstoffatome oder Alkylgruppen mit 1-5 Kohlenstoffatomen darstellen, oder mit einem reaktionsfaehigen Derivat hiervon, falls $R_3'''$ die Carboxyl- oder Hydroxysulfonylgruppe darstellt.

Die Umsetzung wird zweckmaeßigerweise in einem Loesungsmittel oder Loesungsmittelgemisch wie Methylenchlorid, Ethanol, Chloroform, Tetrachlorkohlenstoff, Ether, Tetrahydrofuran, Dioxan, Benzol, Toluol, Acetonitril oder Dimethylformamid, gegebenenfalls in Gegenwart eines die Saeure aktivierenden Mittels oder eines wasserentziehenden Mittels, z.B. in Gegenwart von Chlorameisensaeureethylester, Thionylchlorid, Phosphortrichlorid, Phosphorpentoxid, N'N-Dicyclohexylcarbodiimid/N-Hydroxysuccinimid, N-N'-Carbonyldiimidazol oder N,N'-Thionyldiimidazol oder Triphenylphosphin/Tetrachlorkohlenstoff, oder eines die Hydrazino- oder Aminogruppe aktivierenden Mittels, z.B. Phosphortrichlorid, und gegebenenfalls in Gegenwart einer anorganischen Base wie Natriumcarbonat oder einer tertiaeren organischen Base wie Triethylamin oder Pyridin, welche gleichzeitig als Loesungsmittel dienen koennen, bei Temperaturen zwischen -25 und 250 °C, vorzugsweise jedoch bei Temperaturen zwischen -10 °C und der Siedetemperatur des verwendeten Loesungsmittels durchgefuehrt werden, desweiteren kann waehrend der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Besonders vorteilhaft wird jedoch die Umsetzung in einem entsprechenden Halogenid, z.B. dem Carbonsaeure- oder Sulfonsaeurechlorid, und Hydrazin oder einem entsprechenden Amin, wobei diese gleichzeitig als Loesungsmittel dienen koennen, und bei Temperaturen zwischen 0 und 50 °C durchgefuehrt.

Erhaelt man eine Verbindung der allgemeinen Formel I, in der $R_3$ die Cyangruppe darstellt, so kann diese anschließend mittels Alkoholyse und/oder Hydrolyse in eine entsprechende Verbindung, in der $R_3$ eine Alkoxycarbonylgruppe mit insgesamt 2-5 Kohlenstoffatomen, die Aminocarbonyl- oder Carboxylgruppe und/oder $R_4$ eine Alkoxycarbonylgruppe mit insgesamt 2-4 Kohlenstoffatomen, die Aminocarbonyl- oder Carboxylgruppe darstellen, ueberfuehrt werden und/oder eine Verbindung der allgemeinen Formel I, in der $R_3$ die Carboxygruppe darstellt, mittels Veresterung in eine entsprechende Verbindung der allgemeinen Formel I, in der $R_3$ eine Alkoxycarbonylgruppe mit insgesamt 2-5 Kohlenstoffatomen darstellt, ueberfuehrt werden.

Die nachtraegliche Alkoholyse und/oder Hydrolyse wird zweckmaeßigerweise entweder in Gegenwart einer Saeure wie Salzsaeure, Schwefelsaeure, Phosphorsaeure oder Trichloressigsaeure oder in Gegenwart einer Base wie Natriumhydroxid oder Kaliumhydroxid in einem geeigneten Loesungsmittel wie Wasser, Wasser/Methanol, Ethanol, Wasser/Ethanol, Wasser/Isopropanol oder Wasser/Dioxan bei Temperaturen zwischen -10 und 120 °C, z.B., bei Temperaturen zwischen Raumtemperatur und der Siedetemperatur des Reaktionsgemisches, durchgefuehrt.

Die nachtraegliche Veresterung wird zweckmaeßigerweise in einem geeigneten Loesungsmittel, z.B. in einem entsprechenden Alkohol, Pyridin, Toluol, Methylenchlorid, Tetrahydrofuran oder Dioxan, in Gegenwart eines saeureaktivierenden und/oder wasserentziehenden Mittels wie Thionylchlorid, Chlorameisensaeureethylester, N,N'-Dicyclohexylcarbodiimid oder dessen Isoharnstoffethern, gegebenenfalls in Gegenwart eines Reaktionsbeschleunigers wie Kupfer-chlorid, oder durch Umesterung, z.B. mit einem entsprechenden Kohlensaeurediester, bei Temperaturen zwischen 0 und 100 °C, vorzugsweise jedoch bei Temperaturen zwischen 20 °C und der Siedetemperatur des betreffenden Loesungsmittels, durchgefuehrt.

d) fuer die Umsetzung einer Verbindung der allgemeinen Formel I, in der $R_2$ die Hydroxygruppe, oder X eine Bindung und $R_1$ die Hydroxygruppe, oder in der sowohl $R_1$ als auch $R_2$ eine Hydroxygruppe und X eine Bindung darstellt, zu einer anderen Verbindung der allgemeinen Formel I, in der $R_1$ und/oder $R_2$ die Mercaptogruppe bedeutet.

Die Umsetzung wird nach literaturbekannten Verfahren mit einem das Schwefelatom uebertragenden Reagenz, wie z.B. Phosphorpentasulfid durchgefuehrt, wobei man zweckmaeßigerweise 1-5 Mol $P_2S_5$ vorzugsweise jedoch 1 Mol in einem geeigneten Loesungsmittel umsetzt. Als Loesungsmittel kommen Tetrahydrofuran, Dioxan, Benzol, Toluol oder Pyridin bei Temperaturen zwischen 25 und 125 °C in

Betracht.

Bevorzugt ist jedoch bei einer Raektionsdauer von 1-10 Stunden, vorzugsweise 5 Stunden in Abhaengigkeit vom Reaktionspartner.

e) fuer die Umsetzung einer Verbindung der allgemeinen Formel I, in der $R_2$ eine Aminogruppe bedeutet, oder in der $R_1$ eine Aminogruppe bedeutet (waehrend X eine Bindung darstellt), oder in der X eine Bindung und sowohl $R_1$ als auch $R_2$ eine Aminogruppe darstellen, mit aktivierten Saeurederivaten wie Anhydriden oder Saeurehalogeniden, zu Alkylcarbonylamino- oder Pyridylcarbonylaminoderivaten. Die Umsetzungen werden vorzugsweise in inerten Loesungsmitteln wie Methylenchlorid oder Pyridin bei Temperaturen zwischen 0 ° C und 250 ° C, vorzugsweise jedoch bei der Siedetemperatur des Loesungsmittels durchgefuehrt.

f) fuer die nachtraegliche Alkylierung von Verbindungen der allgemeinen Formel I, in der $R_2$ die Mercaptogruppe, oder in der $R_1$ die Mercaptogruppe und X eine Bindung, oder in der X eine Bindung und sowohl $R_1$ als auch $R_2$ eine Mercaptogruppe darstellen zu den entsprechenden Alkylmercaptoverbindungen. Die Reaktionen werden vorzugsweise in einem Loesungsmittel wie Aceton, Ether, Benzol, Toluol, Dimethylformamid bei Temperaturen zwischen -30 ° C und +100 ° C, vorzugsweise bei Raumtemperatur, in Gegenwart einer Base wie Kaliumcarbonat oder Natriumhydrid und eines Alkylierungsmittels wie Alkylhalogeniden oder Alkylsulfaten durchgefuehrt.

Ferner koennen die erhaltenen Verbindungen der allgemeinen Formel I anschließend gewuenschtenfalls in ihre physiologisch vertraeglichen Saeureadditionssalze mit anorganischen oder organischen Saeuren ueberfuehrt werden. Als Saeuren kommen hierfuer beispielsweise Salzsaeure, Bromwasserstoffsaeure, Schwefelsaeure, Phosphorsaeure, Fumarsaeure, Bernsteinsaeure, Weinsaeure, Zitronensaeure, Milchsaeure, Maleinsaeure oder Methansulfonsaeure in Betracht.

Wie bereits eingangs erwaehnt weisen die neuen Verbindungen der allgemeinen Formel I, deren Tautomere und deren physiologisch vertraegliche Saeureadditionssalze bei einer langen Wirkungsdauer ueberlegene pharmakologische Eigenschaften auf, insbesondere eine blutdrucksenkende und/oder positivinotrope Wirkung und/oder beeinflussen sie die Thrombozytenfunktion und verbessern die Mikrozirkulation.

Zur Herstellung von Arzneimitteln werden die Substanzen der allgemeinen Formel I in an sich bekannnter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die erfindungsgemaeßen neuen Substanzen der allgemeinen Formel I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt.

Derartige Zusaetze sind z.B. Tartrat- und Citratpuffer, Ethanol, Komplexbildner (wie Ethylendiamintetraessigsaeure und deren nicht toxische Salze) und hochmolekulare Polymere (wie fluessiges Polyethylenoxid) zur Viskositaetsregulierung. Feste Traegerstoffe sind z.B. Staerke, Lactose, Mannit, Methylcellulose, Talkum, hochdisperse Kieselsaeuren, hochmolekulare Fettsaeuren (wie Staerinsaeure), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks- und Sueßstoffe enthalten.

Die erfindungsgemaeßen Verbindungen werden ueblicherweise in Mengen von 10-500 mg pro Tag bezogen auf 75 kg Koerpergewicht appliziert. Bevorzugt ist es, 2-3 mal pro Tag 1-2 Tabletten mit einem Wirkstoffgehalt von 5-200 mg zu verabreichen. Die Tabletten koennen auch retardiert sein, wodurch nur noch 1mal pro Tag 1-2 Tabletten mit 10-500 mg Wirkstoff gegeben werden muß. Der Wirkstoff kann auch durch Injektion 1-8mal pro Tag bzw. durch Dauerinfusion gegeben werden, wobei Mengen von 5-200 mg/Tag normalerweise ausreichen.

Bevorzugt im Sinne der vorliegenden Erfindung sind außer den in den Beispielen genannten Verbindungen die folgenden und deren Tautomere.

6-(4-Methoxyphenyl)-1-methyl-3,5-dihydrobenzo[1,2-d: 4,5-d']diimidazol-2-(1H)-on
6-(4-Nitrophenyl)-1-methyl-3,5-dihydrobenzo[1,2-d: 4,5-d']diimidazol-2-(1H)-on
6-(4-Aminophenyl)-1-methyl-3,5-dihydrobenzo[1,2-d: 4,5-d']diimidazol-2-(1H)-on
6-(4-Acetylaminophenyl)-1-methyl-3,5-dihydrobenzo[1,2-d:4,5-d']diimidazol-2-(1H)-on
6-(4-Hydroxyphenyl)-1-methyl-3,5-dihydrobenzo[1,2-d: 4,5-d']diimidazol-2-(1H)-on
6-(4-Chlorphenyl)-1-methyl-3,5-dihydrobenzo[1,2-d: 4,5-d']diimidazol-2-(1H)-on
6-(4-Methylsulfenyl-2-methoxyphenyl)-1-methyl-3,5-dihydrobenzo[1,2-d:4,5-d']diimidazol-2-(1H)-on
6-(4-Methylsulfinyl-2-methoxyphenyl)-1-methyl-3,5-dihydrobenzo[1,2-d:4,5-d']diimidazol-2-(1H)-on
6-(4-Methylsulfonyl-2-methoxyphenyl)-1-methyl-3,5-dihydrobenzo[1,2-d:4,5-d']diimidazol-2-(1H)-on

6-(4-Methylsulfonyloxy-2-methoxyphenyl)-1-methyl-3,5-dihydrobenzo[1,2-d:4,5-d']diimidazol-2-(1H)-on
6-(4-Aminosulfonyl-2-methoxyphenyl)-1-methyl-3,5-dihydrobenzo[1,2-d:4,5-d']diimidazol-2-(1H)-on
6-(4-Methylsulfenylmethyl-2-methoxyphenyl)-1-methyl-3,5-dihydrobenzo[1,2-d:4,5-d']diimidazol-2-(1H)-on
6-(4-Methylsulfinylmethyl-2-methoxyphenyl)-1-methyl-3,5-dihydrobenzo[1,2-d:4,5-d']diimidazol-2-(1H)-on
6-(4-Methylsulfonylmethyl-2-methoxyphenyl)-1-methyl-3,5-dihydrobenzo[1,2-d:4,5-d']diimidazol-2-(1H)-on
6-(N-oxy-4-pyridyl)-1-methyl-3,5-dihydrobenzo[1,2-d: 4,5-d']diimidazol-2-(1H)-on
6-(N-oxy-3-pyridyl)-1-methyl-3,5-dihydrobenzo[1,2-d: 4,5-d']diimidazol-2-(1H)-on
6-(2-Chlor-4-pyridyl)-1-methyl-3,5-dihydrobenzo[1,2-d:4,5-d']diimidazol-2-(1H)-on
6-(2-Methyl-4-pyridyl)-1-methyl-3,5-dihydrobenzo[1,2-d:4,5-d']diimidazol-2-(1H)-on
6-((4-Pyridyl)-2-ethyl)-1-methyl-3,5-dihydrobenzo[1,2-d:4,5-d']diimidazol-2-(1H)-on
6-((4-Pyridyl)-2-ethenyl)-1-methyl-3,5-dihydrobenzo[1,2-d:4,5-d']diimidazol-2-(1H)-on
6-(1,2,5-Thiadiazolyl)-1-methyl-3,5-dihydrobenzo[1,2-d:4,5-d']diimidazol-2-(1H)-on
6-(1,3,4-Thiadiazolyl)-1-methyl-di3,5-dihydrobenzo[1,2-d:4,5-d']diimidazol-2-(1H)-on
6-(2-Pyridyl)-1-ethyl-3,5-dihydrobenzo[1,2-d:4,5-d']diimidazol-2-(1H)-on
6-(N-oxy-4-pyridyl)-1-ethyl-3,5-dihydrobenzo[1,2-d: 4,5-d']diimidazol-2-(1H)-on
6-(3-Pyridazinyl)-1-ethyl-3,5-dihydrobenzo[1,2-d: 4,5-d']diimidazol-2-(1H)-on
6-(1,2,5-Thiadiazol-3-yl)-1-ethyl-3,5-dihydrobenzo[1,2-d:4,5-d']diimidazol-2-(1H)-on
6-(1,3,4-Thiadiazol-2-yl)-1-ethyl-3,5-dihydrobenzo[1,2-d:4,5-d']diimidazol-2-(1H)-on
6-Phenyl-1-ethyl-3,5-dihydrobenzo[1,2-d:4,5-d']diimidazol-2-(1H)-on
6-(2-Dimethylamino-4-nitrophenyl)-1-ethyl-3,5-dihydrobenzo[1,2-d:4,5-d']diimidazol-2-(1H)-on
6-(4-Trifluormethylsulfonyloxy-2-methoxyphenyl)-1-ethyl-3,5-dihydrobenzo[1,2-d:4,5-d']diimidazol-2-(1H)-on
6-(4-(4-Morpholinylsulfonyl)-2-methoxyphenyl)-1-ethyl-3, 5-dihydrobenzo[1,2-d:4,5-d']diimidazol-2-(1H)-on
6-(4-Carboxy-2-methoxyphenyl)-1-ethyl-3,5-dihydrobenzo[1,2-d:4,5-d']diimidazol-2-(1H)-on
6-(4-Ethoxycarbonyl-2-methoxyphenyl)-1-ethyl-3,5-dihydrobenzo[1,2-d:4,5-d']diimidazol-2-(1H)-on
6-(4-Aminocarbonyl-2-methoxyphenyl)-1-ethyl-3,5-dihydrobenzo[1,2-d:4,5-d']diimidazol-2-(1H)-on
6-(4-Cyano-2-methoxyphenyl)-1-ethyl-3,5-dihydrobenzo[1,2-d:4,5-d']diimidazol-2-(1H)-on
6-(3,4,5-Trimethoxyphenyl)-1-ethyl-3,5-dihydrobenzo[1,2-d:4,5-d']diimidazol-2-(1H)-on
6-(2-Furyl)-1-methyl-3,5-dihydrobenzo[1,2-d:4,5-d']diimidazol-2-(1H)-on
6-(2-Thienyl)-1-methyl-3,5-dihydrobenzo[1,2-d:4,5-d']diimidazol-2-(1H)-on
6-(2-Pyrrolyl)-1-methyl-3,5-dihydrobenzo[1,2-d: 4,5-d']diimidazol-2-(1H)-on
6-(4-Imidazolyl)-1-methyl-3,5-dihydrobenzo[1,2-d: 4,5-d']diimidazol-2-(1H)-on
6-(4-Pyrimidinyl)-1-methyl-3,5-dihydrobenzo[1,2-d: 4,5-d']diimidazol-2-(1H)-on
6-(5-Pyrimidinyl)-1-methyl-3,5-dihydrobenzo[1,2-d: 4,5-d']diimidazol-2-(1H)-on
6-(1,2,4-Triazol-3-yl)-1-methyl-3,5-dihydrobenzo[1,2-d:4,5-d']diimidazol-2-(1H)-on
6-(1,2,4,5-Tetrazin-3-yl)-1-methyl-3,5-hydrobenzo[1,2-d:4,5-d']diimidazol-2-(1H)-on
6-(2-Methyl-5-pyrimidinyl)-1-methyl-3,5-dihydrobenzo[1,2-d:4,5-d']diimidazol-2-(1H)-on
6-(2-Hydroxy-5-pyrimidinyl)-1-methyl-3,5-dihydrobenzo[1,2-d:4,5-d']diimidazol-2-(1H)-on
6-(1,2-Dihydro-1-methyl-2-oxo-5-pyrimidinyl)-1-methyl-3,5-dihydrobenzo[1,2-d:4,5-d']diimidazol-2-(1H)-on
6-(6-Methyl-4-pyrimidinyl)-1-methyl-3,5-dihydrobenzo[1,2-d:4,5-d']diimidazol-2-(1H)-on
6-(5,6-Dimethyl-1,2,4-triazin-3-yl)-1-methyl-3,5-dihydrobenzo[1,2-d:4,5-d']diimidazol-2-(1H)-on
6-(3-Methyl-5-pyrazolyl)-1-methyl-3,5-dihydrobenzo[1,2-d:4,5-d']diimidazol-2-(1H)-on
6-(2-Methyl-4-oxazolyl)-1-methyl-3,5-dihydrobenzo[1,2-d:4,5-d']diimidazol-2-(1H)-on
6-(2-Pyrrolyl)-1-propyl-3,5-dihydrobenzo[1,2-d:4,5-d']diimidazol-2-(1H)-on
6-(3-Pyridyl)-1-propyl-3,5-dihydrobenzo[1,2-d:4,5-d']diimidazol-2-(1H)-on
6-Phenyl-1-propyl-3,5-dihydrobenzo[1,2-d:4,5-d']diimidazol-2-(1H)-on
6-(4-Methylaminocarbonylamino-2-methoxyphenyl)-1-propyl-3,5-dihydrobenzo[1,2-d:4,5-d']diimidazol-2-(1H)-on
6-(4-Aminocarbonylamino-2-methoxyphenyl)-1-propyl-3,5-dihydrobenzo[1,2-d:4,5-d']diimidazol-2-(1H)-on
6-(4-Methylaminocarbonylamino-2-methoxyphenyl)-1-propyl-3,5-dihydrobenzo[1,2-d:4,5-d']diimidazol-2-(1H)-on
6-(4-Pyridyl)-1-methyl-3,5-dihydrobenzo[1,2-d:4,5-d']diimidazol-2-(1H)-thion
6-(4-Pyridyl)-2-methyl-1,5-dihydrobenzo[1,2-d:4,5-d']diimidazol
6-(4-Pyridyl)-1,2-dimethyl-1,5-dihydrobenzo[1,2-d:4,5-d']diimidazol
6-(4-Pyridyl)-5H-imidazo-[4,5-f]benzoxazol
6-(5-Pyrimidinyl)-2,3-dihydro-5H-imidazo[4,5-f]benzoxazol-2-on
6-(2-Chlor-4-pyridyl)-2,3-dihydro-5H-imidazo[4,5-f]benzoxazol-2-on
6-(4-Cyanophenyl)-2,3-dihydro-5H-imidazol[4,5-f]benzoxazol-2-on
6-(1,2,5-Thiadiazolyl)-2,3-dihydro-5H-imidazo[4,5-f]benzoxazol-2-on

6-(1,3,4-Thiadiazolyl)-2,3-dihydro-5H-imidazo[4,5-f]benzoxazol-2-on
6-(4-Pyridyl)-2,3-dihydro-5H-imidazo[4,5-f]benzoxazol-2-thion
6-(4-Pyridyl)-2-methyl-5H-imidazo[4,5-f]benzoxazol
6-(4-Pyridyl)-5H-imidazo[4,5-f]benzthiazol
6-(4-Pyridyl)-2,3-dihydro-5H-imidazo[4,5-f]benzthiazol-2-thion
6-(3-Pyridyl)-2-methyl-5H-imidazo[4,5-f]benzthiazol
6-(5-Isoxazolyl)-1-methyl-3,5-dihydrobenzo[1,2-d: 4,5-d']diimidazol-2-(1H)-on
6-(5-Isothiazolyl)-1-methyl-3,5-dihydrobenzo[1,2-d: 4,5-d,]diimidazol-2-(1H)-on
6-(5-Isoxazolyl)-2,3-dihydro-5H-imidazo[4,5-f]benzoxazol-2-on
6-(5-Isothiazolyl)-2,3-dihydro-5H-imidazo[4,5-f]benzoxazol-2-on
6-(5-Isoxazolyl)-2,3-dihydro-5H-imidazo[4,5-f]benzothiazol-2-on
6-(5-Isothiazolyl)-2,3-dihydro-5H-imidazo[4,5-f]benzothiazol-2-on
6-(1,2,5-Thiadiazol-3-yl)-2,3-dihydro-5H-imidazo[4,5-f]benzothiazol-2-on

Neue Verbindungen der allgemeinen Formel IX, X und XI sind außer den in den Beispielen genannten die folgenden

6-Nitro-5-(4-pyridylcarbonylamino)benzoxazolinon
6-Nitro-5-(3-pyridylcarbonylamino)benzoxazolinon
6-Nitro-5-phenylcarbonylamino-benzoxazolinon
6-Nitro-5-(2-thienylcarbonylamino)benzoxazolinon
6-Nitro-5-(2-furylcarbonylamino)benzoxazolinon
6-Nitro-5-(4-pyridazinylcarbonylamino)benzoxazolinon
5-Nitro-6-(4-pyridylcarbonylamino)benzoxazolinon
5-Nitro-6-(3-pyridylcarbonylamino)benzoxazolinon
5-Nitro-6-phenylcarbonylamino-benzoxazolinon
5-Nitro-6-(2-methoxyphenyl-carbonylamino)benzoxazolinon
5-Nitro-6-(2-methylmercaptophenyl-carbonylamino)benzoxazolinon
5-Nitro-6-(4-cyanophenyl-carbonylamino)benzoxazolinon
5-Nitro-6-(2-furyl-carbonylamino)benzoxazolinon
5-Nitro-6-(2-thienyl-carbonylamino)benzoxazolinon
5-Nitro-6-(3-thienyl-carbonylamino)-benzoxazolinon
5-Nitro-6-(4-pyridazinyl-carbonylamino)benzoxazolinon
5-Nitro-6-(5-pyrimidinyl-carbonylamino)benzoxazolinon
5-Nitro-6-(5-pyrazinyl-carbonylamino)benzoxazolinon
5-Nitro-6-(2-methyl-4-pyridinyl-carbonylamino)benzoxazolinon
5-Nitro-6-(2-chlor-4-pyridinyl-carbonylamino)benzoxazolinon
7-Brom-2-methyl-6-(4-pyridyl-carbonylamino)benzoxazol
7-Brom-2-methyl-6-(3-pyridyl-carbonylamino)benzoxazol
7-Brom-2-methyl-6-phenylcarbonylamino-benzoxazol
7-Brom-2-methyl-6-(2-methoxyphenyl-carbonylamino)benzoxazol
7-Brom-2-methyl-6-(2-methylmercaptophenyl-carbonylamino)benzoxazol
7-Brom-2-methyl-6-(4-cyanophenyl-carbonylamino)benzoxazol
7-Brom-2-methyl-6-(3-pyridyl-carbonylamino)benzthiazol
7-Brom-2-methyl-6-phenylcarbonylamino-benzthiazol
7-Brom-2-methyl-6-(2-thienyl-carbonylamino)benzthiazol
7-Brom-2-methyl-6-(3-thienyl-carbonylamino)benzthiazol
7-Brom-2-methyl-6-(2-furyl-carbonylamino)benzthiazol
7-Brom-2-methyl-6-(4-pyridazinyl-carbonylamino)benzthiazol
7-Brom-2-methyl-6-(5-pyrimidinyl-carbonylamino)benzthiazol
7-Brom-2-methyl-6-(5-pyrazinyl-carbonylamino)benzthiazol
7-Brom-2-methyl-6-(2-methyl-4-pyridinylcarbonylamino)benzthiazol
7-Brom-2-methyl-6-(2-methoxyphenyl-carbonylamino)benzthiazol
7-Brom-2-methyl-6-(2-methylmercaptophenyl-carbonylamino)benzthiazol
7-Brom-6-(4-pyridyl-carbonylamino)benzoxazol
7-Brom-6-(4-pyridyl-carbonylamino)benzthiazol

Beispiel 1

2-(4-Pyridyl)-1,5-dihydrobenzo[1,2-d:4,5-d']diimidazol

14.8 g (100 mmol) 5,6-Diaminobenzimidazol, 10.7 g (100 mmol) Pyridin-4-aldehyd und 5 ml konzentrierte Salzsaeure in 500 ml Ethanol werden eine Stunde unter Rueckfluß zum Sieden erhitzt. Nach Klaeren der Loesung mit Aktivkohle wird eingeengt und der Rueckstand mit wenig Wasser digeriert. Nach Absaugen und Trocknen erhaelt man 6.98 g (29 %) der Titelverbindung als Hydrochlorid. Fp. >280 °C, farblose Kristalle.

Beispiel 2

6-(4-Pyridyl)-1-methyl-1,5-dihydrobenzo[1,2-d:4,5-d']diimidazol

a) Zu einer Loesung von 16.0 g (76.9 mmol) 5,6-Dinitrobenzimidazol in 500 ml DMF werden 80 ml 1 M Natriummethylatloesung gegeben und im Vakuum auf ca. 150 ml eingeengt. Nach Zugabe von 15 ml (241 mmol) Methyliodid wird 1 Tag bei 40 °C geruehrt. Die Loesung gießt man auf 1.5 Liter Eiswasser. Der ausgefallene Niederschlag wird abgesaugt und getrocknet.

10 g (58 %) 1-Methyl-5,6-dinitrobenzimidazol, Fp. 238-240 °C farblose Kristalle.

b) 4.40 g (20.0 mmol) 1-Methyl-5,6-dinitrobenzimidazol und 0.5 g Adams Katalysator in 250 ml Ethanol werden 4 Stunden bei 40 °C mit 4 bar $H_2$ Druck hydriert. Der Katalysator wird abfiltriert und das Filtrat nach Zusatz von 2.10 g (20.0 mmol) Pyridin-4-aldehyd und 3 ml konzentrierter Salzsaeure unter Rueckfluß zum Sieden erhitzt. Nach chromatographischer Reinigung (Kieselgel), Essigester-Methanol 1:1, v/v) ergibt Einengen der entsprechenden Fraktionen 1.1 g (21 %) farblose Kristalle mit Fp. >280 °C (Semihydrochlorid der Titelverbindung).

Beispiel 3

Analog dem Beispiel 2 erhaelt man 6-(4-Hydroxy-3,5-dimethoxyphenyl)-1-methyl-3,5-dihydrobenzo[1,2-d:4,5-d']diimidazol als Hydrochlorid mit Fp. >280 °C.

Beispiel 4

6-(3-Pyridyl)-1-methyl-3,5-dihydrobenzo[1,2-d:4,5-d']diimidazol-2-(1H)-on

a) 2.20 g (9.2 mmol) 5,6-Dinitro-1-methyl-benzimidazol-2-on wurde in Gegenwart von 0.44 g Platindioxid in 730 ml Methanol bei 60 °C und 100 bar drei Stunden lang hydriert. Die so erhaltenen Loesung von 5,6-Diamino-1-methylbenzimidazol-2-on wurde nach Filtration ohne weitere Reinigung eingesetzt.

b) 0.98 g (9.2 mmol) Pyridin-3-aldehyd und 73 mg p-Toluolsulfonsaeure wurden vorgelegt und die unter a) hergestellte Loesung dazugegeben. Man erhitzte zwei Stunden unter Rueckfluß, entfernte das Loesungsmittel im Vakuum, nahm den Rueckstand in Wasser auf und neutralisierte mit 2 N Ammoniakloesung. Der ausgefallene Niederschlag wurde abgesaugt und aus Isopropanol kristallisiert. Man erhielt 450 mg (20 %) der Titelverbindung mit Fp. >300 °C.

Mit analog Beispiel 4 a erhaltenen Loesungen von 5,6-Diamino-1-methyl-benzimidazol-2-on und den angegebenen Aldehyden in Gegenwart katalytischer Mengen der angegebenen Saeuren wurden analog Beispiel 4 b die folgenden Verbindungen dargestellt:

| | Bezeichnung | Ausbeute [%] | Schmp. [°C] Reinigung |
|---|---|---|---|
| 1. | 6-(4-Pyridyl)-1-methyl-3,5-dihydrobenzo[1,2-d:4,5-d']-diimidazol-2-(1H)-on<br>aus<br>Pyridin-4-aldehyd in Gegenwart von konz. Salzsaeure | 35 | >320°C Saeulenchromatographisch (Kieselgel; $CH_2Cl_2$:$CH_3OH$= 19:1) |
| 2. | 6-Phenyl-1-methyl-3,5-dihydrobenzo[1,2-d:4,5-d']diimdazol-2-(1H)-on<br>aus<br>Benzaldehyd in Gegenwart von p-Toluolsulfonsaeure | 11 | >300°C Kristallisation aus i-Propanol |
| 3. | 6-(4-Dimethylamino-phenyl)-1-methyl-3,5-dihydrobenzo[1,2-d:4,5-d']diimidazol-2-(1H)-on<br>aus<br>p-Dimethylaminobenzaldehyd in Gegenwart von p-Toluolsulfonsaeure | 20 | 238-241°C zweimal umkristall. aus Methanol |

| | Bezeichnung | Ausbeute [%] | Schmp. [°C] Reinigung |
|---|---|---|---|
| 4. | 6-(4-(1-Imedazolyl)-phenyl)-1-methyl-3,5-dihydrobenzo-[1,2-d:4,5-d']diimidazol-2-(1H)-on<br><br>aus<br><br>4-(1-Imidazolyl)-benzaldehyd in Gegenwart von p-Toluolsulfonsaeure | 7 | 260°C Zers. Kristallisation aus Essigester |
| 5. | 6-(4-Diethylamino-2-methoxy-phenyl)-1-methyl-3,5-dihydrobenzo[1,2-d:4,5-d']diimidazol-2-(1H)-on<br><br>aus<br><br>4-Diethylamino-2-methoxy-benzaldehyd in Gegenwart von p-Toluolsulfonsaeure | 36 | >300°C Kristallisation aus Methanol/Dichlormethan |
| 6. | 6-(4-Pyridyl)-1-ethyl-3,5-dihydrobenzo[1,2-d:4,5-d']-diimidazol-2-(1H)-on<br><br>aus<br><br>5,6-Diamino-1-ethyl-benzimidazol-2-on und Pyridyl-4-aldehyd in Gegenwart von p-Toluolsulfonsaeure | 28 | 237-239°C Kristallisation aus Essigester |

Weiterhin wurden analog Beispiel 4 dargestellt:

| | Bezeichnung | Ausbeute [%] | Schmp. [°C] Reinigung |
|---|---|---|---|
| 7. | 6-(4-Pyridyl)-1-(1-propyl)-3,5-dihydrobenzo[1,2-d:4,5-d']di-imidazol-2-(1H)-on<br>aus<br>5,6-Diamino-1-(1-propyl)-benz-imidazol-2-on<br>und<br>Pyridyl-4-aldehyd<br>in Gegenwart von<br>p-Toluolsulfonsaeure | 21 | 235-237<br>Kristallisation aus Essigester |
| 8. | 6-(2-Furyl)-2,3-dihydro-5H-imidazo-[4,5-f]benzoxazol-2-on<br>aus<br>5,6-Diamino-benzoxazolin-2-on<br>und<br>Furfural<br>in Gegenwart von<br>p-Toluolsulfonsaeure | 30 | 282-285<br>Kristallisation aus Isopropanol |

| | Bezeichnung | Ausbeute [%] | Schmp. [°C] Reinigung |
|---|---|---|---|
| 9. | 6-(2-Thienyl)-2,3-dihydro-5H-imidazo-[4,5-f]benzoxazol-2-on aus 5,6-Diamino-benzoxazolin-2-on und Thiophen-2-aldehyd in Gegenwart von Essigsaeure | 14 | 270-274°C Saeulenchromatographie: (Kieselgel; Dichlormethan: Methanol=15:1) |
| 10. | 6-(3-Thienyl)-2,3-dihydro-5H-imidazo-[4,5-f]benzoxazol-2-on aus 5,6-Diamino-benzoxazolin-2-on und Thiophen-3-aldehyd in Gegenwart von Essigsaeure | 21 | 295-300°C Kristallisation aus Dioxan/Wasser |
| 11. | 6-(4-Pyridyl)-2,3-dihydro-5H-imidazo-[4,5-f]benzthiazol-2-on aus 5,6-Diamino-benzthiazolin-2-on und Pyridyl-4-aldehyd in Gegenwart von Essigsaeure | 36 | >300°C Mit Methanol aus der Huelse extrahieren, den Rueckstand in 2 N NaOH loesen, mit Eisessig ansaeuern, absaugen. |

Beispiel 5

6-(4-Pyridazinyl)-2,3-dihydro-5H-imidazo[4,5-f]benzoxazol-2-on

a) 3.35 g (17.2 mmol) 6-Amino-5-nitrobenzoxazolinon werden in 120 ml Methanol in Gegenwart von 0.5 g 10 % Palladium auf Kohle bei Normaldruck hydriert. Nach 1 h wird der Katalysator abfiltriert und die

Loesung im Vakuum eingeengt. Der Rueckstand wird ohne Reinigung weiter umgesetzt.

b) Eine Mischung aus 0.5 g 5,6-Diamino-benzoxazolin-2-on und 0.41 g Pyridazin-4-carbonsaeure mit 5.4 g warmer Polyphosphorsaeure hielt man eine Stunde in einem auf 150 ° C vorgeheiztem Bad, loeste den Ansatz nach dem Erkalten in 30 ml Eiswasser, neutralisierte mit waessriger Ammoniakloesung, saugte die erhaltene Faellung ab, wusch mit Wasser und erhielt so 0.38 g Rohprodukt. Dieses suspendierte man in Wasser, gab bis zur klaren Loesung waessrige Kalilauge zu, behandelte mit Aktivkohle, saugte ab, neutralisierte das Filtrat, saugte die erhaltene Faellung ab, wusch mit Wasser und erhielt so 0.22 g der Titelverbindung als Monohydrat mit Schmp. >330 ° C.

Analog dem Beispiel 5 erhielt man die folgenden Verbindungen:

| | Bezeichnung | Ausbeute [%] | Schmp. [°C] Reinigung |
|---|---|---|---|
| 1. | 6-Pyrazinyl-2,3-dihydro-5H-imidazo-[4,5-f]benzoxazol-2-on aus 5,6-Diaminobenzoxazolin-2-on und Pyrazincarbonsaeure | 35 | 335°C In Pyridin mit Aktivkohle behandeln. |
| 2. | 6-(4-Aminocarbonyl-phenyl)-2,3-dihydro-5H-imidazo[4,5-f]benzoxazol-2-on aus 5,6-Diaminobenzoxazolin-2-on und 4-Cyanobenzoesaeure | 30 | >330°C In Ethanol mit Aktivkohle behandeln. |
| 3. | 6-(2-Methoxyphenyl)-2,3-dihydro-5H-imidazo[4,5-f]benzoxazol-2-on aus 5,6-Diaminobenzoxazolin-2-on und 2-Methoxybenzoesaeure | 39 | 319-321°C Kieselgelsaeule: $CH_2Cl_2$: methanol.$NH_3$ = 10:1 |

| | Bezeichnung | Ausbeute [%] | Schmp. [°C] Reinigung |
|---|---|---|---|
| 4. | 6-(2-Chlor-3-pyridyl)-2,3-dihydro-5H-imidazo[4,5-f]benzoxazol-2-on aus 5,6-Diaminobenzoxazolin-2-on und 2-Chlornicotinsaeure | 8 | 310°C (Zers.) Kieselgel-saeule Laufmittel: Butanol/Essigsaeure 80:20; Kristallisation aus Methanol/Ether |
| 5. | 6-(2-Methylmercapto-phenyl)-2,3-dihydro-5H-imidazo[4,5-f]benzoxazol-2-on aus 5,6-Diaminobenzoxazolin-2-on und 2-Methylmercaptobenzoesaeure | 36 | 208-211°C Kieselgel-saeule, Laufmittel: Essigester |
| 6. | 6-(4-Pyridazinyl)-1-methyl-3,5-dihydrobenzo[1,2-d:4,5-d']-diimidazol-2-(1H)-on aus 5,6-Diamino-1-methyl-benzimidazol-2-on (s. Beisp. 4) und Pyridazin-4-carbonsaeure | 14 | >300°C Kristallisation aus Methanol/Dichlormethan |

| | Bezeichnung | Ausbeute [%] | Schmp. [°C] Reinigung |
|---|---|---|---|
| 7. | 6-(4-Pyridazinyl)-1-ethyl-3,5-dihydrobenzo[1,2-d:4,5-d']-diimidazol-2-(1H)-on<br>aus<br>5,6-Diamino-1-ethyl-benzimidazol-2-on<br>und<br>Pyridazin-4-carbonsaeure | 14 | >300°C<br>Kristallisation aus Pyridin |
| 8. | 6-(4-Pyridylmethyl)-1-methyl-3,5-dihydrobenzo[1,2-d:4,5-d']-diimidazol-2-(1H)-on<br>aus<br>5,6-Diamino-1-methyl-benzimidazol-2-on<br>und<br>4-Pyridylessigsaeure | 48 | >300°C<br>Kristallisation aus Methanol |

Beispiel 6

6-Phenyl-2,3-dihydro-5H-imidazo[4,5-f]benzoxazol-2-on

1 g rohes 5,6-Diamino-benzoxazolin-2-on (erhalten durch Hydrierung des 6-Amino-5-nitro-benzoxazolinons analog Beisp. 5 a) suspendierte man in 30 ml Methylenchlorid und 1.82 g Triethylamin, tropfte bei Eiskuehlung rasch 1.26 g Benzoylchlorid zu, ließ die entstandene Loesung 15 min bei 10°C nachruehren, tropfte erneut bei ca. 0°C 0.91 g Triethylamin und dann 0.5 ml Benzoylchlorid zu, ließ 30 min bei 10°C und 15 min bei Raumtemp. nachruehren, dampfte dann im Vacuum ein, rieb den Eindampfrueckstand mit Eiswasser an (ca. 30 ml) und erhielt so 2.2 g festes rohes Produkt, welches ohne weitere Reinigung in der folgenden Reaktionsstufe eingesetzt wurde.

1.9 g davon kochte man mit 82 ml Ethanol und 11 ml konz. HCl 5 h unter Rueckfluß, gab dann erneut 11 ml konz. Salzsaeure zu, kochte weitere 4 h unter Rueckfluß, saugte nach dem Erkalten ueber Nacht das erhaltene Kristallisat ab, wusch mit Ethanol und Ether und erhielt so 0.62 g HCl-Salz der Titelverbindung; Fp. ca. 350°C, Zers.

0.1 g davon lieferte nach Neutralisation mit waessrigem Ammoniak in waesseriger Suspension (bis etwa pH 9) 0.08 g der Titelverbindung als freie Base mit 1 mol $H_2O$ pro mol der gewuenschten Substanz, Fp. 298/300°C.

Analog Beispiel 6 erhielt man die folgenden Verbindungen:

| | Bezeichnung | Ausbeute [%] | Schmp. [°C] Reinigung |
|---|---|---|---|
| 1. | 6-(4-Pyridyl)-2,3-dihydro-5H-imidazo[4,5-f]benzoxazol-2-on aus 5,6-Diamino-benzoxazolin-2-on und 4-Pyridincarbonylchlorid-HCl | 51 | >360°C Kristallisation aus Dimethylformamid |
| 2. | 6-(3-Pyridyl)-2,3-dihydro-5H-imidazo[4,5-f]benzoxazol-2-on aus 5,6-Diamino-benzoxazolin-2-on und 3-Pyridincarbonylchlorid.HCl | 28 | >360 Kristallisation aus Wasser/ Methanol |
| 3. | 6-(2-Methyl-4-pyridyl)-2,3-dihydro-5H-imidazo[4,5-f]-benzoxazol-2-on aus 5,6-Diamino-benzoxazolin-2-on und 2-Methyl-4-pyridincarbonyl-chlorid.HCl | 11 | >300°C Zers. Kieselgelsaeule, Laufmittel: Butanol, Essigsaeure, Wasser |

| | Bezeichnung | Ausbeute [%] | Schmp. [°C] Reinigung |
|---|---|---|---|
| 4. | 6-(1-Propyl)-2,3-dihydro-5H-imidazo-[4,5-f]benzoxazol-2-on aus 5,6-Diamino-benzoxazolin-2-on und n-Buttersaeureanhydrid | 34 | 177-180° Kristallisation aus Essigester |
| 5. | 6-Trifluormethyl-2,3-dihydro-5H-imidazo[4,5-f]benzoxazol-2-on aus 5,6-Diamino-benzoxazolin-2-on und Trifluoressigsaeureanhydrid | 45 | 348-350°C Kristallisation aus Wasser |

Beispiel 7

6-Methyl-2,3-dihydro-5H-imidazo[4,5-f]benzoxazol-2-on

a) 4.9 g 6-Amino-benzoxazolin-2-on (Fp. 190/192 °C) suspendierte man in 112 ml Acetanhydrid, ruehrte 3 h bei Raumtemp., goß dann den Ansatz in 150 ml Eiswasser, ruehrte 30 min nach, saugte ab, wusch mit Wasser und erhielt so 5.7 g 6-Acetamido-benzoxazolinon-2 vom Fp. 330/332 °C (Aufsch.).

b) 2.85 g davon trug man portionsweise in 17 ml konz. $H_2SO_4$ ein, ruehrte ca. 1.5 h bis Lsg. eintrat. Bei -10 °C tropfte man dazu eine Lsg. von 0.65 ml $HNO_3$ (D 1.51) in 5 ml konz. $H_2SO_4$ von 0 °C, ruehrte 5 min bei -10 °C weiter, goß dann in 100 ml Wasser + Eis, saugte nach 30 min die ungeloeste Substanz ab, wusch mit $H_2O$ und erhielt so 3 g 6-Acetamido-5-nitrobenzoxazolin-2-on vom Fp. 213 °C, nach Umkristallisation aus 300 ml Methanol war der Fp. 244/246 °C. Man erhielt so insgesamt 2.7 g.

c) 0.95 g davon in 280 ml Methanol geloest (warm) hydrierte man mit 0.32 g Pd-C-Katalysator (10proz.) 1 h bei Raumtemp., saugte dann ab, wusch mit Methanol, dampfte das Filtrat im Vac. ein und erhielt so 0.7 g 6-Acetamido-5-amino-benzoxazolin-2-on, Fp. >320 °C.

Diese so erhaltenen 0.7 g suspendierte man in 14 ml Eisessig, brachte den Ansatz in ein 100 °C warmes Bad, hielt 1 h diese Temperatur, dampfte dann im Vacuum ein, loeste den Eindampfrueckstand in 40 ml Wasser bei ca. 50 °C, behandelte mit Kohle, neutralisierte mit Ammoniak bis ca. pH 8, saugte nach 20 min Stehen ab, wusch mit Wasser und erhielt so 0.45 g der Titelverbindung , Fp. 355 °C, Zers..

Analog dem Beispiel 7 erhielt man aus 5-Acetylamino-6-nitro-1-methyl-benzimidazol-2-on mit 66 % Ausbeute 1,6-Dimethyl-3,5-dihydrobenzo[1,2-d:4,5-d']diimidazol-2-(1H)-on mit dem Fp. >300 °C nach Kristallisation aus Isopropanol.

Beispiel 8

2-Methyl-6-(4-pyridyl)-5H-imidazo[4,5-f]benzthiazol

a) 13.2 g (80.0 mmol) 6-Amino-2-methylbenzthiazol und 22.2 ml (160 mmol) Triethylamin werden in 120

ml Dichlormethan geloest. Unter Eiskuehlung werden 14.3 g (80.0 mmol) 4-Pyridincarbonylchlorid-Hydrochlorid zugesetzt. Das Loesungsmittel wird im Vacuum entfernt, der Rueckstand mit Wasser digeriert. Nach Kristallisation aus Ethanol erhaelt man 17.0 g (79 %) farblose Kristalle mit Fp. 209-210 °C.

b) 15.9 g (59.0 mmol) 2-Methyl-6-(4-pyridyl-carbonylamino)benzthiazol werden in 60 ml Eisessig geloest. Nach Zugabe von 5 ml Wasser tropft man 3.4 ml (65.0 mmol) Brom zu. Nach Zugabe von 18.4 g (59.0 mmol) Silbersulfat ruehrt man 2 h bei Raumtemperatur. Das Silbersulfat wird abfiltriert und mit wenig Eisessig gewaschen. Das Filtrat wird mit Ammoniak neutralisiert und der ausgefallene Niederschlag abgesaugt. Nach Kristallisation aus Ethanol erhaelt man 12.5 g (61 %) farblose Kristalle mit Fp. 194-196 °C.

c) 12.5 g (36.0 mmol) 7-Brom-2-methyl-6-(4-pyridylcarbonylamino)benzthiazol werden in eine -10 °C kalte Mischung aus 1.7 ml (40.0 mmol) rauchender Salpetersaeure und 30 ml konzentrierter Schwefelsaeure unter Ruehren eingetragen. Nach 8 h Ruehren bei Raumtemperatur gießt man die Loesung auf Eiswasser und saugt den Niederschlag ab. Nach Kristallisation aus Ethanol erhaelt man 7.3 g (52 %) Kristalle mit Fp. 251-253 °C.

d) 5.0 g (12.7 mmol) 7-Brom-2-methyl-5-nitro-6-(4-pyridylcarbonylamino)benzthiazol und 2.1 ml (15.2 mmol) Triethylamin werden in 200 ml Methanol geloest. In Gegenwart von 1.0 g 10 % Palladium auf Kohle hydriert man bei Normaldruck und Raumtemperatur. Nach Filtration des Katalysators und Entfernen des Loesungsmittels im Vacuum versetzt man den Rueckstand mit 4 ml konzentrierter Salzsaeure und ruehrt 3 Stunden bei 60 °C. Man neutralisiert die Loesung mit 2 N Natronlauge, saugt den Niederschlag ab und reinigt ueber eine kurze Kieselgelsaeule.
Nach Kristallisation aus Isopropanol erhaelt man 2.55 g (75 %) der Titelverbindung mit Fp. >300 °C.

Beispiel 9

6-(4-Pyridyl)-3,5-dihydrobenzo[1,2-d:4,5-d']diimidazol-2-(1H)-on

a) 10.0 g (59.0 mmol) Nitro-2,4,5-triaminobenzol und 20 ml Triethylamin werden in 500 ml Dichlormethan geloest und 11.6 g (65.0 mmol) Pyridin-4-carbonylchlorid-hydrochlorid portionsweise zugesetzt. Nach 5 h Ruehren bei Raumtemperatur wird das Loesungsmittel im Vacuum entfernt und der Rueckstand mit Wasser digeriert. Der Rueckstand wird in 500 ml Ethanol und 80 ml konzentrierter Salzsaeure 36 h unter Rueckfluß zum Sieden erhitzt. Das Loesungsmittel wird im Vacuum entfernt, der Rueckstand mit waessriger Ammoniakloesung digeriert und abgesaugt. Man erhaelt 12.0 g (80 %) 5-Amino-6-nitro-2-(4-pyridyl)-benzimidazol, das ohne weitere Reinigung eingesetzt wird.

b) 5 g des obigen Rueckstandes wird in 300 ml Methanol in Gegenwart von 0.4 g 10 % Palladium auf Kohle bei Normaldruck und Raumtemperatur hydriert. Der Katalysator wird abgesaugt, das Filtrat im Vacuum zur Trockene eingeengt und der Rueckstand (3 g 5,6-Diamino-2-(4-pyridyl)-benzimidazol)ohne weitere Reinigung eingesetzt.

c) 3.00 g (13.3 mmol) des obigen Rueckstandes werden in 120 ml 2 N Salzsaeure geloest und Phosgen durch die Loesung geleitet. Der ausgefallene Niederschlag wird mit waessriger Ammoniakloesung digeriert und abgesaugt. Nach Kristallisation aus Methanol/Dichlormethan (4:1 v/v) erhaelt man 0.72 g (22 %) der Titelverbindung mit Fp. >300 °C. Analog dem Beispiel 9 c erhaelt man aus 3.5 g 5,6-Diamino-1-methyl-benzimidazolin-2-on in 70 ml 2 N Salzsaeure nach vierstuendigem Einleiten von Phosgen und Stehen bei Raumtemperatur ueber Nacht 1-Methyl-1,2,3,5,6,7-hexahydro[1,2-d: 4,5-d']diimidazol-2,6-dion mit Fp. >300 °C.

Beispiel 10

2,3,5,6-Tetrahydro-7H-imidazo[4,5-f]benzoxazol-2,6-dion

3.3 g (0.02 mmol) 5,6-Diaminobenzoxaiolin-2-on und 5.5 g (0.034 mol) 1,1'-Carbonyldiimidazol loeste man in 150 ml Dioxan und erhitzte drei Stunden zum Sieden. Nach Abkuehlen auf Raumtemperatur saugte man die braeunliche kristalline Substanz ab, kochte mit Dimethylformamid aus, saugte ab, und wusch mit Methanol. Man erhielt 65 % der Titelverbindung mit Fp. >360 °C.

Beispiel 11

6-(N-Oxy-4-pyridyl)-1,2-dihydro-5H-imidazo[4,5-f]benzoxazol-2-on

1.9 g (7.5 mmol) 6-(4-Pyridyl)-1,2-dihydro-5H-imidazo[4,5-f]benzoxazol-2-on suspendierte man in 20 ml Eisessig und gab 6 ml 30 % $H_2O_2$ zu. Nach drei Wochen ruehrte man die Suspension in Wasser ein und saugte den Niederschlag ab. Die Reinigung erfolgte durch Saeulenchromatographie an Kieselgel mit einem Butanol-Essigsaeure-Wasser-Gemisch. Die entsprechenden Fraktionen wurden im Vakuum eingeengt, der Rueckstand in 2 N Natronlauge geloest, ungeloeste Bestandteile filtriert und die Loesung mit Salzsaeure angesaeuert. Der ausgefallene Niederschlag wurde abgesaugt, mit Wasser gewaschen und bei 120 °C getrocknet. Man erhielt 0.6 g (30 %) der Titelverbindung mit Fp. >360 °C.

Beispiel 12

2-Amino-6-(4-pyridyl)-1,5-dihydrobenzo[1,2-d:4,5-d']diimidazol

2.9 g 5,6-Diamino-2-(4-pyridyl)benzimidazol und 1.3 g Bromcyan wurden unter Feuchtigkeitsausschluß zwei Stunden bei 85 °C geruehrt. Nach Abkuehlen auf Raumtemperatur wurde mit Aktivkohle behandelt, filtriert und das Filtrat zur Trockene eingeengt. Den Rueckstand suspendierte man in Wasser, stellte mit waessriger Ammoniakloesung einen pH-Wert von 8.5 ein und saugte ab. Der Rueckstand (1.47 g) zog man auf 15 g Kieselgel 60 auf, gab auf eine Saeule mit 150 Kieselgel 60 und eluierte mit einer Mischung aus Methylenechlorid und ammoniakalischem Methanol (95:5 bis 92:8). Die entsprechenden Fraktionen wurden zur Trockene eingeengt und der Rueckstand in Methanol geloest. Man gab Isopropanol bis zur Truebung zu und ließ Kristallisieren. Man erhielt 0.52 g der Titelverbindung mit dem Fp. >320 °C.

Beispiel 13

2-Acetylamino-6-(4-pyridyl)-1,5-dihydrobenzo[1,2-d:4,5-d']diimidazol

210 mg der in Beispiel 12 erhaltenen Verbindung ruehrte man vier Tage bei 50 °C in 20 ml Essigsaeureanhydrid. Man versetzt mit 7 mg Natriumacetat und destillierte nach zwei Stunden im Hochvacuum zur Trockene. Der Rueckstand (160 mg) wurde saeulenchromatographisch gereinigt (Kieselgel; Methylenchlorid:ammoniakal. Methanol = 98:2 bis 92:8). Die entsprechenden Fraktionen wurden vereinigt und zur Trockene destilliert. Der Rueckstand wurde aus Methanol kristallisiert. Man erhielt 105 mg der Titelverbindung mit dem Fp. > 330 °C.

**Patentansprüche**

1. Benzimidazole der Formel I

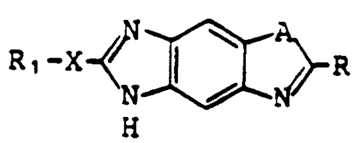

(I),

in welcher

$R_1$ den Pyrrol-, Furan-, Thiophen-, Pyrazol-, Imidazol-, Thiazo Isothiazol-, Oxazol-, Isoxazol-, Triazol-, Tetrazol-, Thiadiazol-, Oxadiazol-, Pyridin-, N-Oxy-Pyridin-, Pyrazin-, N,N-Dioxypyrazin-, Pyrimidin-, N,N-Dioxypyrimidin-, Pyridazin-, Oxazin-, Thiazin-, Triazin-, Tetrazinrest darstellt, sowie deren $C_1$-$C_6$-Alkyl-, $C_1$-$C_6$-Alkoxy-, $C_1$-$C_6$-Alkylmercapto-, und chlorsubstituierten Derivate,
oder den Phenylrest der allgemeinen Formel II,

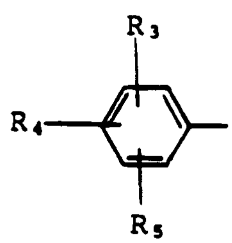

(II),

in der

$R_3$ Wasserstoff, eine Alkylsulfonyloxy-, Trifluormethylsulfonyloxy-, Alkylsulfenylmethyl, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino oder Dialkylaminogruppe substituierte Carbonylgruppe oder eine durch eine Amino-, Dialkylamino- oder Morpholinogruppe substituierte Sulfonylgruppe, wobei jeder der vorstehend genannten Alkylteile 1 oder 2 Kohlenstoffatome enthalten kann, eine Nitro-, Cyan- oder Alkylaminosulfonylgruppe mit 1-4 Kohlenstoffatomen, eine Alkylcarbonylamino-, Aminocarbonylamino- oder N-Alkyl-aminocarbonylaminogruppe, eine Alkylmercapto-, Alkylsulfinyl- oder Alkylsulfonylgruppe, wobei jeder der vorgenannten Alkylteile 1 oder 2 Kohlenstoffatome enthalten kann, eine Halogen-, Amino-, Hydroxy-, Dialkylamino-, Alkyl-, Alkoxy-, Alkenyloxy- oder Alkinyloxygruppe vorzugsweise mit 1-3 Kohlenstoffatomen, eine Cyanmethoxy- oder Methoxycarbonylmethoxygruppe, die Trifluormethylgruppe oder die 1-Imidazolylgruppe,

$R_4$ Wasserstoff, eine Alkylgruppe mit 1-3 Kohlenstoffatomen, eine Alkoxy- oder Dialkylaminogruppe mit 1 oder 2 Kohlenstoffatomen in jedem Alkylteil oder ein Halogenatom, und

$R_5$ Wasserstoff oder die Methoxygruppe ist,

X ein Valenzstrich, eine $C_1$-$C_4$-Alkylengruppe oder die Vinylengruppe sein kann,

A ein Sauerstoffatom, ein Schwefelatom, oder die Gruppe N-$R_6$ bedeutet, wobei $R_6$ ein Wasserstoffatom oder eine $C_1$-$C_4$-Alkylgruppe darstellt,

$R_2$ ein Wasserstoffatom, eine $C_1$-$C_4$-Alkyl-, eine Hydroxy- oder eine Mercaptogruppe darstellt, mit Ausnahme der Verbindung 2-(4-Aminophenyl)-1,7-dihydrobenzo-[1,2-d:4,5-d']bis imidazol,

deren Tautomere und deren physiologisch vertraegliche Salze anorganischer und organischer Saeuren.

2. Benzimidazole der Formel I gemaeß Anspruch 1, in welcher

$R_1$ den Pyrrol-, Furan-, Thiophen-, Pyrazol-, Imidazol-, Thiazo Isothiazol-, Oxazol-, Isoxazol-, Triazol-, Tetrazol-, Thiadiazol-, Oxadiazol-, Pyridin-, N-Oxy-Pyridin-, Pyrazin-, N,N-Dioxypyrazin-, Pyrimidin-, N,N-Dioxypyrimidin-, Pyridazin-, Oxazin-, Thiazin-, Triazin-, Tetrazinrest darstellt, sowie deren Methyl-, Ethyl-, Methoxy-, Ethoxy-, Methylmercapto-, Ethylmercapto- und chlorsubstituierten Derivate,
oder den Phenylrest der allgemeinen Formel II, in der

$R_3$ ein Wasserstoff, die Methansulfonyloxy-, Trifluormethansulfonyloxy-, Methansulfonylamino-, Trifluormethansulfonylamino-, Methansulfonyl-methylamino-, Trifluormethansulfonylmethylamino-, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfonyl-, Hydroxy-, Methyl-, Methoxy-, Propargyloxy-, Cyanmethoxy-, Methoxycarbonylmethoxy-, Cyan-, Chlor-, Nitro-, Amino-, Dimethylamino-, Trifluormethyl-, oder die 1-Imidazolylgruppe darstellt,

$R_4$ Wasserstoff, die Methyl-, Methoxy-, Dimethylamino- oder Chlorgruppe bedeutet und

$R_5$ Wasserstoff oder die Methoxygruppe ist,

X einen Valenzstrich, eine Alkylengruppe mit 1 oder 2 Kohlenstoffatomen oder die Vinylengruppe bedeutet,

A ein Sauerstoff- oder Schwefelatom oder die Gruppe $NR_6$ ist, in der $R_6$ Wasserstoff, die Methyl-, Ethyl-, Propyl-, Isopropylgruppe ist,

$R_2$ Wasserstoff, eine Methyl-, Ethyl-, Propyl-, Butyl-, Isopropyl-, 2-Butyl-, tert.-Butyl-, Hydroxy- oder Mercaptogruppe ist,

deren Tautomere und deren physiologisch vertraegliche Salze anorganischer oder organischer Saeuren.

3. Benzimidazole der Formel I gemaeß Anspruch 1 oder 2, in welcher

$R_1$ den Pyridin-, N-Oxy-Pyridin-, Pyridazin-, Pyrimidin-, Pyrazin-, Furan- oder Thiophen-Rest, der gegebenenfalls durch Alkyl und/oder Halogen substituiert sein kann, Phenyl, das gegebenenfalls ein- oder zweifach durch Methoxy, Methylmercapto, Aminocarbonyl, Dimethylamino, Diethylamino, 1-Imidazolyl und/oder Hydroxy substituiert sein kann,

X einen Valenzstrich, die Methylen- oder Vinylengruppe ist,

A ein Sauerstoff- oder Schwefelatom oder die Gruppe $NR_6$ ist, in der $R_6$ Wasserstoff, Methyl, Ethyl oder n-Propyl ist, und

$R_2$ ein Wasserstoffatom, eine Hydroxy- oder Methylgruppe darstellt,
deren Tautomere und deren physiologisch vertraegliche Salze anorganischer und organischer

Saeuren.

4. Verfahren zur Herstellung der Benzimidazole der Formel I gemäß einem der Ansprüche 1-3, dadurch gekennzeichnet, daß man in an sich bekannter Weise
a) eine Verbindung der Formel X

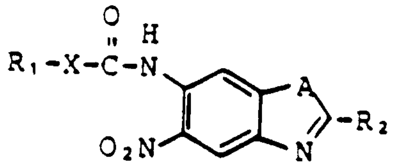

(X)

oder der Formel XI

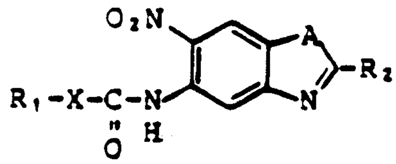

(XI)

oder der Formel IX

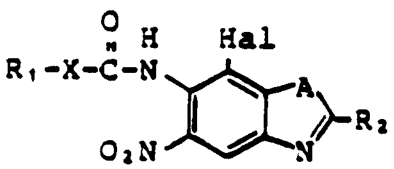

(IX)

in den $R_1$, X, A, $R_2$ die oben angegebenen Bedeutungen besitzen und Hal ein Halogenatom bedeutet, reduziert und anschließend cyclisiert, oder
b) eine Verbindung der Formel XII

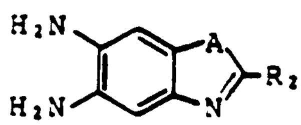

(XII),

in der A und $R_2$ die oben angegebenen Bedeutungen haben, mit einer Verbindung der Formel VIII

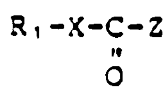

(VIII),

in der $R_1$ und X die oben angegebenen Bedeutungen besitzen und Z Wasserstoff oder einen reaktiven Rest darstellt, oder
c) eine Verbindung der allgemeinen Formel XIII

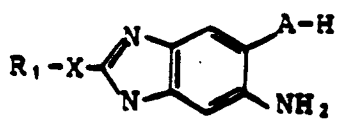

(XIII),

in der $R_1$, X und A die oben angegebenen Bedeutungen besitzen, mit entsprechenden Säurehalogeniden

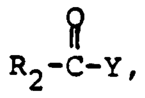 (XIV),

in denen $R_2$ die oben angegebene Bedeutung hat und Y einen leicht abspaltbaren Rest darstellt, umsetzt und anschließend cyclisiert, anschließend gewuenschtenfalls erhaltene Verbindungen der allgemeinen Formel I in andere Verbindungen der Formel I ueberfuehrt, sowie gewuenschtenfalls die Verbindungen mit anorganischen oder organischen Saeuren in physiologisch vertraegliche Salze ueberfuehrt.

**5.** Arzneimittel, enthaltend eine oder mehrere Verbindungen gemaeß Anspruch 1, 2 oder 3 neben pharmazeutisch geeigneten Traeger- und/oder Hilfsstoffen.

**6.** Verwendung von Verbindungen gemaeß Anspruch 1, 2 oder 3 zur Herstellung von Arzneimitteln zur Prophylaxe und Behandlung von Herz- und Kreislauferkrankungen.

**7.** Verbindungen der Formel X

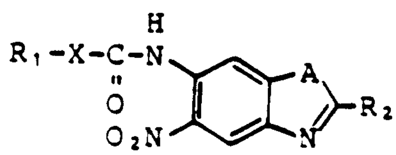 (X)

in welcher
$R_1$, $R_2$ und A die Bedeutungen gemäß einem der Ansprüche 1-3 besitzen, sowie deren Tautomere.

**8.** Verbindungen der Formel XI

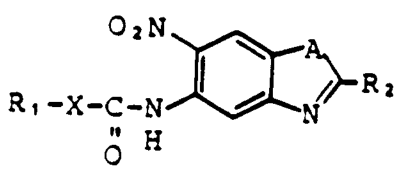 (XI)

in welcher $R^1$, $R^2$ und A die Bedeutungen gemäß einem der Ansprüche 1-3 besitzen, sowie deren Tautomere.

**9.** Verbindungen der Formel IX

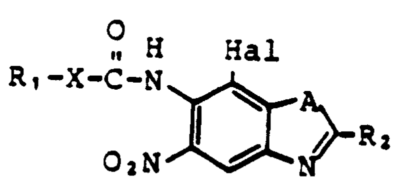 (IX)

in welcher $R_1$, $R_2$ und A die Bedeutungen gemäß einem der Ansprüche 1-3 besitzen, und
Hal eine Halogenidgruppe darstellt,
sowie deren Tautomere.

**10.** Die Verbindungen
6-(1-Propyl)-2,3-dihydro-5H-imidazo[4,5-f]benzoxazol-2-on
6-Trifluormethyl-2,3-dihydro-5H-imidazo[4,5-f]benzoxazol-2-on
6-Methyl-2,3-dihydro-5H-imidazo[4,5-f]benzoxazol-2-on
1,6-Dimethyl-2,3-dihydro-imidazo[4,5-f]benzimidazol-2-on
6-Hydroxy-1-methyl-2,3-dihydro-imidazo[4,5-f]benzimidazol-2-on
6-Hydroxy-2,3-dihydro-imidazo[4,5-f]benzoxazol-2-on

## Claims

**1.** Benzimidazoles of the formula I

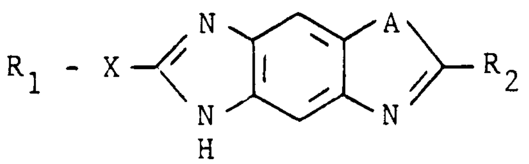

(I)

in which $R_1$ represents the pyrrole, furan, thiophene, pyrazole, imidazole, thiazole, isothiazole, oxazole, isoxazole, triazole, tetrazole, thiadiazole, oxadiazole, pyridine, N-oxypyridine, pyrazine, N,N-dioxypyrazine, pyrimidine, N,N-dioxypyrimidine, pyridazine, oxazine, thiazine, triazine, tetrazine radical, as well as their $C_1$-$C_6$-alkyl-, $C_1$-$C_6$-alkoxy-, $C_1$-$C_6$-alkylmercapto- and chloro-substituted derivatives, or the phenyl radical of the general formula II

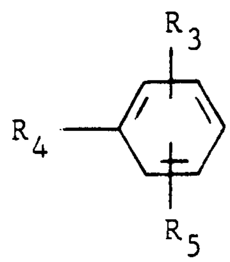

(II)

in which $R_3$ is hydrogen, an alkylsulphonyloxy, trifluoromethylsulphonyloxy, alkylsulphenylmethyl, alkylsulphinylmethyl, alkylsulphonylmethyl, alkylsulphonylamino, N-alkyl-alkylsulphonylamino, trifluoromethylsulphonylamino or N-alkyl-trifluoromethylsulphonylamino group, a carbonyl group substituted by a hydroxyl, alkoxy, amino, alkylamino or dialkylamino group or a sulphonyl group substituted by an amino, dialkylamino or morpholino group, whereby each of the above-mentioned alkyl moieties can contain 1 or 2 carbon atoms, a nitro, cyano or alkylaminosulphonyl group with 1 - 4 carbon atoms, an alkylcarbonylamino, aminocarbonylamino or N-alkylaminocarbonylamino group, an alkylmercapto, alkylsulphinyl or alkylsulphonyl group, whereby each of the above-mentioned alkyl moieties can contain 1 or 2 carbon atoms, a halogen, amino, hydroxyl, dialkylamino, alkyl, alkoxy, alkenyloxy or alkynyloxy group, preferably with 1 - 3 carbon atoms, a cyanomethoxy or methoxycarbonylmethoxy group, the trifluoromethyl group or the 1-imidazolyl group, $R_4$ hydrogen, an alkyl group with 1 - 3 carbon atoms, an alkoxy or dialkylamino group with 1 or 2 carbon atoms in each alkyl moiety or a halogen atom, $R_5$ hydrogen or the methoxy group, X can be a valency bond, a $C_1$-$C_4$-alkylene group or the vinylene group, A signifies an oxygen atom, a sulphur atom or the group N-$R_6$, whereby $R_6$ represents a hydrogen atom or a $C_1$-$C_4$-alkyl group, $R_2$ represents a hydrogen atom, a $C_1$-$C_4$-alkyl, a hydroxyl or a mercapto group, with the exception of the compound 2-(4-aminophenyl)-1,7-dihydrobenzo[1,2-d;4,5-d']-bis-imidazole, their tautomers and their physiologically compatible salts of inorganic and organic acids.

**2.** Benzimidazoles of the formula I according to claim 1, in which $R_1$ represents the pyrrole, furan, thiophene, pyrazole, imidazole, thiazole, isothiazole, oxazole, isoxazole, triazole, tetrazole, thiadiazole, oxadiazole, pyridine, N-oxypyridine, pyrazine, N,N-dioxypyrazine, pyrimidine, N,N-dioxypyrimidine, pyridazine, oxazine, thiazine, triazine, tetrazine radical, as well as their methyl-, ethyl-, methoxy-, ethoxy-, methylmercapto-, ethylmercapto- and chlorosubstituted derivatives, or the phenyl radical of the

general formula II, in which $R_3$ represents a hydrogen, the methanesulphonyloxy, trifluoromethanesulphonyloxy, methanesulphonylamino, trifluoromethanesulphonylamino, methanesulphonylmethylamino, trifluoromethanesulphonylmethylamino, methylsulphenylmethyl, methylsulphinylmethyl, methylsulphonylmethyl, aminocarbonyl, aminosulphonyl, methylaminosulphonyl, dimethylaminosulphonyl, acetylamino, methylmercapto, methylsulphonyl, hydroxyl, methyl, methoxy, propargyloxy, cyanomethoxy, methoxycarbonylmethoxy, cyano, chloro, nitro, amino, dimethylamino, trifluoromethyl or the 1-imidazolyl group, $R_4$ signifies hydrogen, the methyl, methoxy, dimethylamino or chloro group and $R_5$ is hydrogen or the methoxy group, X is a valency bond, an alkylene group with 1 or 2 carbon atoms or the vinylene group, A is an oxygen or sulphur atom or the group $NR_6$, in which $R_6$ is hydrogen, the methyl, ethyl, propyl, isopropyl group, $R_2$ is hydrogen, a methyl, ethyl, propyl, butyl, isopropyl, 2-butyl, tert.-butyl, hydroxyl or mercapto group, their tautomers and their physiologically compatible salts of inorganic and organic acids.

3. Benzimidazoles of the formula I according to claim 1 or 2 in which $R_1$ is the pyridine, N-oxypyridine, pyridazine, pyrimidine, pyrazine, furan or thiophene radical, which can possibly be substituted by alkyl and/or halogen, phenyl which can possibly be substituted once or twice by methoxy, methylmercapto, aminocarbonyl, dimethylamino, diethylamino, 1-imidazolyl and/or hydroxyl, X is a valency bond, the methylene or vinylene group, A is an oxygen or sulphur atom or the group $NR_6$, in which $R_6$ is hydrogen, methyl, ethyl or n-propyl, and $R_2$ represents a hydrogen atom, a hydroxyl or methyl group, their tautomers and their physiologically compatible salts of inorganic and organic acids.

4. Process for the preparation of benzimidazoles of the formula I according to one of claims 1 - 3, characterised in that in per se known way one

a) reduces a compound of the formula X

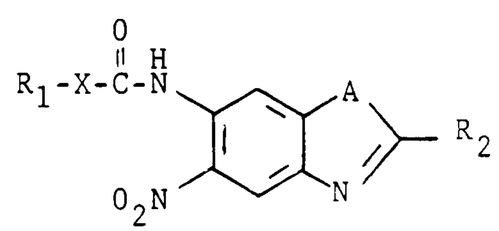

(X)

or of the formula XI

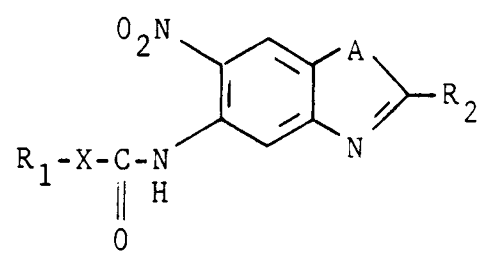

(XI)

or of the formula IX

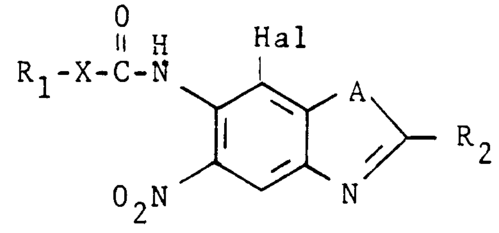

(IX)

in which $R_1$, X, A, $R_2$ possess the above-given meanings, and subsequently cyclises, or
b) reacts a compound of the formula XII

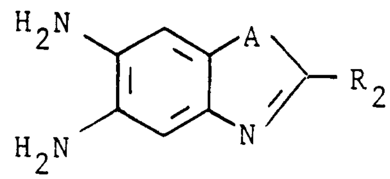

(XII)

in which A and $R_2$ have the above-given meanings, with a compound of the formula VIII

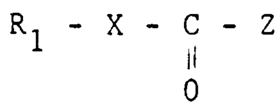

(VIII)

in which $R_1$ and X possess the above-given meanings and Z represents hydrogen or a reactive residue, or
c) reacts a compound of the general formula XIII

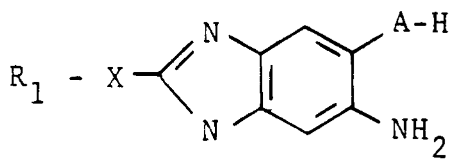

(XIII)

in which $R_1$, X and A possess the above-given meanings, with corresponding acid halides

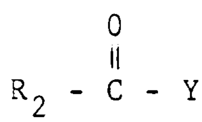

(XIV),

in which $R_2$ has the above-given meaning and Y represents a residue which is easily split off, and subsequently cyclises,
subsequently, if desired, converts the compounds obtained of the general formula I into other compounds of the formula I, as well as, if desired, converts the compounds with inorganic or organic acids into physiologically compatible salts.

**5.** Medicaments containing one or more compounds according to claim 1, 2 or 3, besides pharmaceutically suitable carrier and/or adjuvant materials.

**6.** Use of compounds according to claim 1, 2 or 3 for the preparation of medicaments for the prophylaxis and treatment of heart and circulatory diseases.

**7.** Compounds of the formula X

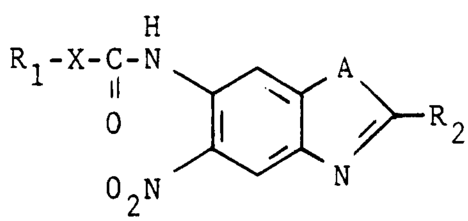

(X)

in which $R_1$, $R_2$ and A possess the meanings according to one of claims 1 - 3, as well as their tautomers.

**8.** Compounds of the formula XI

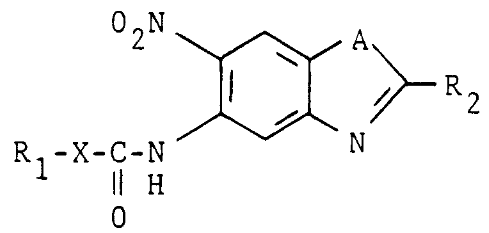

(XI)

in which $R_1$, $R_2$ and A possess the meanings according to one of claims 1 - 3, as well as their tautomers.

**9.** Compounds of the formula IX

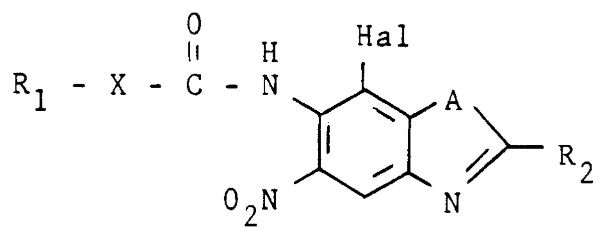

(IX)

in which $R_1$, $R_2$ and A possess the meanings according to one of claims 1 - 3 and Hal represents a halide group, as well as their tautomers.

**10.** The compounds
6-(1-propyl)-2,3-dihydro-5H-imidazo[4,5-f]benzoxazol-2-one,
6-trifluoromethyl-2,3-dihydro-5H-imidazo[4,5-f]benzoxazol-2-one,
6-methyl-2,3-dihydro-5H-imidazo[4,5-f]benzoxazol-2-one,
1,6-dimethyl-2,3-dihydroimidazo[4,5-f]benzimidazol-2-one,
6-hydroxy-1-methyl-2,3-dihydroimidazo[4,5-f]benzimidazol-2-one,
6-hydroxy-2,3-dihydroimidazo[4,5-f]benzoxazol-2-one.

## Revendications

**1.** Benzimidazole de la formule I :

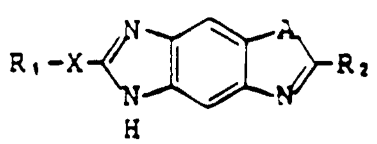

(I),

dans lequel

$R_1$ représente le radical pyrrole, furanne, thiophène, pyrazole, imidazole, thiazole, isothiazole, oxazole, isoxazole, triazole, tétrazole, thiadiazole, oxadiazole, pyridine, N-oxy-Pyridine, Pyrazine, N,N-dioxy-pyrazine, pyrimidine, N,N-dioxypyrimidine, pyridazine, oxazine, thiazine, triazine, tétrazine, $C_1$-$C_6$-Alkylo, $C_1$-$C_6$-alkoxy, $C_1$-$C_6$-alkylmercapto, de même que leurs dérivés de substitution chlorés ou le radical phényle de la formule générale II

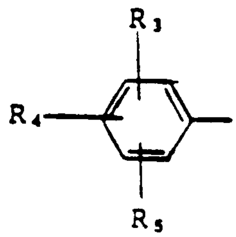

(II),

dans laquelle

$R_3$ est un hydrogène, un groupe d'alkylsulfonyloxy, trifluorméthylsulfonyloxy, alkylsulfenylméthyle, alkylsulfinylméthyle, alkylsulfonylméthyle, alkylsulfonylamino, N-alkyl-alkylsulfonylamino, trifluorméthylsulfonylamino ou un groupe carbonyle substitué choisi dans le groupe N-alkyl-trifluormethylsulfonylamino, hydroxy, alkoxy, amino, alkyloamino ou dialkylamino ou un groupe sulphonyle substitué choisi dans le groupe amino, dialkylamino ou morpholino d'où chaque élément alkyle précédemment indiqué peut contenir 1 ou 2 atomes de carbone, un groupe nitro, cyan ou alkylaminosulfonyle comportant 1 à 4 atomes de carbone, un groupe alkylcarbonylamino, aminocarbonylamino ou N-alkylaminocarbonylamino, un groupe d'alkylmercapto, alkylsulfinyle ou alkylsulfonyle, où chacun des éléments alkyles précédemment indiqués peut contenir 1 ou 2 atomes de carbone, un groupe halogène, amino, hydroxy, dialkylamino, alkyle, alkoxy, alkényloxy ou alkinyloxy, comportant de préférence 1 à 3 atomes de carbone, un groupe cyanméthoxy ou méthoxycarbonylméthoxy, le groupe trifluorméthyle ou le groupe 1-imidazolyle,

$R_4$ est de l'hydrogène, un groupe comportant 1 à 3 atomes de carbone, un groupe alkoxy ou dialkylamino comportant 1 ou 2 atomes de carbone dans chaque élément alkyle ou un atome halogène,

$R_5$ de l'hydrogène ou le groupe méthoxy,

X peut être une valence, un groupe alkyle $C_1$-$C_4$ ou le groupe vinyle,

A un atome d'oxygène, un atome de soufre ou le groupe N-$R_6$, où $R_6$ représente un atome d'hydrogène ou un groupe alkyle $C_1$-$C_4$,

$R_2$ représente un atome d'hydrogène, un groupe alkyle $C_1$-$C_4$, un groupe hydroxy ou un groupe mercapto à l'exception du composé 2-(4-aminophényle)-1,7-dihydrobenzo-(1,2-d:4,5-d')bis imidazole, leurs tautomères et sels physiologiques compatibles d'acides minéraux et organiques.

**2.** Benzimidazole de la formule I selon la revendication 1, dans lequel

$R_1$ représente le radical pyrrole, furanne, thiophène, pyrazole, imidazole, thiazole, isothiazole, oxazole, isoxazole, triazole, tétrazole, thiadiazole, oxadiazole, pyridine, N-oxy-Pyridine, Pyrazine, N,N-dioxy-pyrazine, pyrimidine, N,N-dioxypyrimidine, pyridazine, oxazine, thiazine, triazine, tétrazine, de même que méthyle, éthyle, méthoxy, éthoxy, méthylmercapto, éthylmercapto et leurs dérivés de substitution chlorés

ou le radical phényle de la formule générale II dans laquelle

$R_3$ représente un hydrogène, le groupe de méthansulfonyloxy, trifluorméthansulfonyloxy, méthansulfonylamino, trifluorméthansulfonylamino, méthansulfonyl-méthylamino, trifluorméthansulfonylméthylamino, méthylsulfenylméthyle, méthylsulfinylméthyle, méthylsulfonylméthyle, aminocarbonyle, aminosulfonyle, méthylaminosulfonyle, diméthylaminosulfonyle, acétylamino méthylmercapto, méthylsulfonyle, hydroxy, méthyle, méthoxy, propargyloxy, cyanméthoxy, méthoxycarbonylmétoxy, cyan, chlore, nitro, amino, diméthylamino, trifluorméthyle ou le groupe 1-imidazolyle,

$R_4$ désigne l'hydrogène, le groupe méthyle, métoxy, diméthylamino ou chlore, et

$R_5$ est de l'hydrogène ou le groupe méthoxy,

X représente une valence, un groupe alkyle comportant 1 ou ou 2 atomes de carbone ou le groupe vinyle,

A est un atome d'oxygène ou de soufre ou le groupe $NR_6$, dans $R_6$ on trouve l'hydrogène, le groupe méthyle, éthyle, propyle, isopropyle,

$R_2$ est de l'hydrogène, un groupe méthyle, éthyle, propyle, butyle, isopropyle, 2-butyle, 3-butyle, hydroxy ou mercapto

leurs tautomères et sels physiologiques compatibles d'acides minéraux et organiques.

**3.** Benzimidazole de la formule I selon la revendication 1 ou 2, dans lequel

$R_1$ représente le radical pyridine, N-oxy-pyridine, Pyridazine, pyrimidine, pyrazine, furanne ou thiozène qui peut être substitué par un alkyle et/ou un halogène, le radical phényle qui peut être monosubstitué ou bisubstitué par métoxy, méthylmercapto, aminocarbonyle, diméthylamino, diéthylamino, 1-imidazolyle et/ou hydroxy,

X représente une valence, le groupe méthyle ou vinyle,

A est un atome d'oxygène ou de soufre ou le groupe $NR_6$, dans $R_6$ on trouve l'hydrogène, le groupe méthyle, éthyle ou n-propyle, et

$R_2$ représente un atome d'hydrogène, un groupe hydroxy ou méthyle, leurs tautomères et sels physiologiques compatibles d'acides minéraux et organiques.

**4.** Procédé pour la préparation de la benzimidazole de la formule I selon lune quelconque des revendications 1 à 3, caractérisé en ce que, d'une manière connue en soi, on réduit puis on cyclise :

a) un composé de la formule X

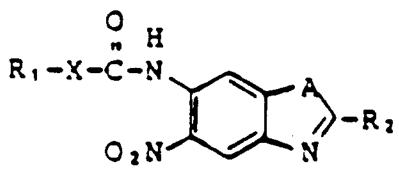

(X)

ou de la formule XI

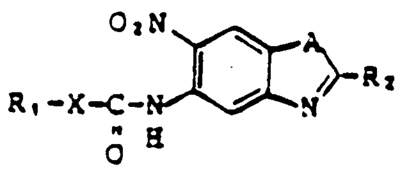

(XI)

ou de la formule IX

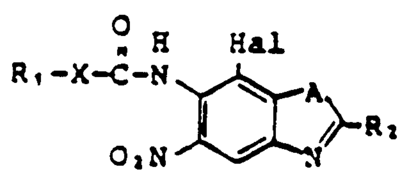

(IX)

dans laquelle R, X, A, $R_2$ ont la même signification que précédemment et où Hal désigne un atome d'halogène ou on transforme puis, on cyclise

b) un composé de la formule XII

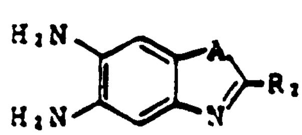

(XII),

dans lequel A et R ont la même signification que précédemment avec un composé de la formule VIII

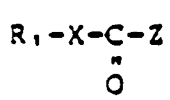

(VIII),

dans lequel $R_1$ et X ont la même signification que précédemment et où Z représente de l'hydrogène ou un radical réactivé,

c) un composé de la formule générale XIII

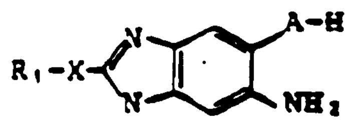

(XIII) ,

dans lequel $R_1$, X et A ont la même signification que précédemment avec les halogénures d'acide correspondants

$$R_2-\overset{\overset{O}{\|}}{C}-Y,$$

(XIV) ,

dans lesquels $R_2$ a la même signification que précédemment et où Y représente un radical qui peut être facilement éliminé, et

on convertit ensuite, si on le souhaite, le composé obtenu de la formule générale I en un autre composé de la formule I, de même que l'on convertit, si on le souhaite, le composé comportant des acides minéraux ou organiques en sels physiologiques compatibles.

**5.** Produit médicamenteux contenant un ou plusieurs composés selon la revendication 1, 2 ou 3 en plus de supports pharmaceutiques et/ou d'adjuvants.

**6.** Utilisation des composés selon la revendication 1, 2 ou 3 pour la préparation de produits médicamenteux destinés à la prophylaxie et au traitement de maladies cardiovasculaires.

**7.** Composés de la formule X

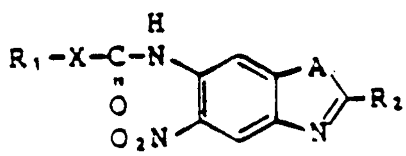

(X)

dans lesquels

$R_1$, $R_2$ et A ont la même signification que dans l'une quelconque des revendications 1 à 3, de même que leurs tautomères.

**8.** Composés de la formule XI

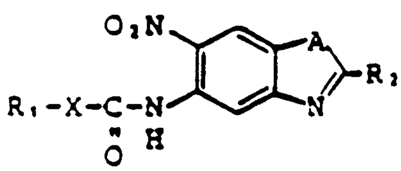

(XI)

dans lesquels $R_1$, $R_2$ et A ont la même signification que dans l'une quelconque des revendications 1 à 3, de même que leurs tautomères.

**9.** Composés de la formule IX

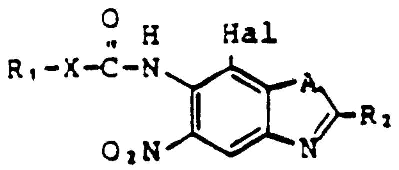

(IX)

dans lesquels $R_1$, $R_2$ et A ont la même signification que dans l'une quelconque des revendications 1 à 3 et où Hal représente un groupe d'halogénures de même que leurs tautomères.

**10.** Les composés :
6-(1-propyle)-2,3-dihydro-5H-imidazo(4,5-f)benzoxazol-2-on
6-trifluorméthyle-2,3-dihydro-5H-imidazo(4,5-f)benzoxazol-2-on
6-méthyle-2,3-dihydro-5H-imidazo(4,5-f)benzoxazol-2-on
1,6-diméthyle-2,3-dihydro-imidazo(4,5-f)benzimidazol-2-on
6-hydroxy-1-méthyle-2,3-dihydro-imidazo(4,5-f)benzimidazol-2-on
6-hydroxy-2,3-dihydro-imidazo(4,5-f)benzoxazol-2-on